(19) Europäisches Patentamt
European Patent Office
Office européen des brevets

(11) EP 1 281 963 A2

(12) EUROPEAN PATENT APPLICATION

(43) Date of publication:
05.02.2003 Bulletin 2003/06

(21) Application number: 02016401.8

(22) Date of filing: 22.07.2002

(51) Int Cl.[7]: **G01N 33/542**, G01N 33/58, G01N 33/68, C12Q 1/48, C12N 15/62

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: 30.07.2001 EP 01402064

(71) Applicant: WARNER-LAMBERT COMPANY
Morris Plains, New Jersey 07950 (US)

(72) Inventor: De Mendez, Isabelle
75014 Paris (FR)

(74) Representative:
Dufresne, Guillaume Alain François et al
Pfizer SCA,
Fresnes Laboratories
3-9, rue de la Loge
B.P. 100
94265 Fresnes Cédex (FR)

(54) **Method for the screening of compounds that inhibit the interaction between a proline-rich peptide and a SH3 domain comprising peptide**

(57) The present invention relates to a method for the screening of compounds of therapeutical value which possess the ability of inhibiting the interaction between a proline-rich peptide and a SH3 domain-comprising peptide, which compounds are able to selectively target proteins involved in a variety of intracellular signalling pathways.

EP 1 281 963 A2

## SEQUENCE LISTING

<110> Warner Lambert Company

<120> Method for the screening of compounds that inhibit the interaction between a proline-rich peptide and a SH3 domain-comprising peptide

<130> HTRF-SH3 Domains - Warner Lambert

<140>
<141>

<160> 4

<170> PatentIn Ver. 2.1

<210> 1
<211> 81
<212> ADN
<213> Homo sapiens

<400> 1
cggaggcacc atcaagcagc cgcccagcaa ccccccgccg cggcccccgg ccgaggcccg 60
caagaagccc agcgaggagg a 81

<210> 2
<211> 402
<212> ADN
<213> Homo sapiens

<400> 2
gacatcaccg gccccatcat cctgcagacg taccgcgcca ttgccgacta cgagaagacc 60
tcgggctccg agatggctct gtccacgggg gacgtggtgg aggtcgtgga gaagagcgag 120
agcggttggt ggttctgtca gatgaaagca aagcgaggct ggatcccagc atccttcctc 180
gagcccctgg acagtcctga cgagacggaa gaccctgagc ccaactatgc aggtgagcca 240
tacgtcgcca tcaaggccta cactgctgtg gagggggacg aggtgtccct gctcgagggt 300
gaagctgttg aggtcattca caagctcctg gacggctggt gggtcatcag gaaagacgac 360
gtcacaggct actttccgtc catgtacctg caaaagtcgg gg 402

<210> 3
<211> 28
<212> PRT
<213> Homo sapiens

<400> 3
Cys Gly Gly Thr Ile Lys Gln Pro Pro Ser Asn Pro Pro Pro Arg Pro
1 5 10 15

Pro Ala Glu Ala Arg Lys Lys Pro Ser Glu Glu Glu
20 25

<210> 4
<211> 134
<212> PRT
<213> Homo sapiens

```
<400> 4
Asp Ile Thr Gly Pro Ile Ile Leu Gln Thr Tyr Arg Ala Ile Ala Asp
 1               5                  10                  15

Tyr Glu Lys Thr Ser Gly Ser Glu Met Ala Leu Ser Thr Gly Asp Val
            20                  25                  30

Val Glu Val Val Glu Lys Ser Glu Ser Gly Trp Trp Phe Cys Gln Met
        35                  40                  45

Lys Ala Lys Arg Gly Trp Ile Pro Ala Ser Phe Leu Glu Pro Leu Asp
    50                  55                  60

Ser Pro Asp Glu Thr Glu Asp Pro Glu Pro Asn Tyr Ala Gly Glu Pro
65                  70                  75                  80

Tyr Val Ala Ile Lys Ala Tyr Thr Ala Val Glu Gly Asp Glu Val Ser
                85                  90                  95

Leu Leu Glu Gly Glu Ala Val Glu Val Ile His Lys Leu Leu Asp Gly
            100                 105                 110

Trp Trp Val Ile Arg Lys Asp Asp Val Thr Gly Tyr Phe Pro Ser Met
        115                 120                 125

Tyr Leu Gln Lys Ser Gly
    130
```

## Description

### FIELD OF INVENTION

The present invention relates to a method for the screening of compounds of therapeutical value which possess the ability of inhibiting the interaction between a proline-rich peptide and a SH3 domain-comprising peptide, which compounds are able to selectively target proteins involved in a variety of intracellular signalling pathways.
The invention also deals with reagents that are specifically designed for carrying out such a screening method.

### BACKGROUND OF THE INVENTION

Signal transduction refers to the transmission of extracellular information, in a coordinated, specially defined manner, to intracellular targets. Information transfer occurs primarily through changes in molecular interactions or covalent modifications of proteins, peptides, lipids and other small molecules. This leads to a variety of events including allosteric modulation of enzyme activity, formation of multimolecular assemblies or changes in intracellular localisation. The result is often a net increase in the local concentration of primed molecules capable of further propagating the signal to additional downstream effectors.
The majority of molecular interactions in signalling pathways are governed by a small set of conserved, non-catalytic protein domains. These modules, which include SH2, SH3, WW, PDZ, PTB and EH domains, each recognise a specific peptide motif in their targets.
SH3 (Src homology 3) domains are small non-catalytic protein modules that mediate protein-protein interactions by binding to proline-rich peptide sequences. SH3 domains are typically 55 to 70 aminoacids in length. SH3 domains have been found in numerous intracellular proteins. More than 50 SH3 domains are known, and these SH3 domains are widely distributed , having been found in kinases, lipases, GTPases, adapter proteins, structural proteins as well as viral regulatory proteins.
Protein-protein interactions involving SH3 domains occur in transient associations between proteins of eukaryotic signal transduction pathways resulting in the formation of complex multiprotein aggregates. Formation of these aggregates leads to enzymatic modifications of the complexes, resulting in the production of new protein-protein interaction sites and propagation and amplification of the intracellular chemical signal.
SH3 domains also mediate interactions that regulate enzyme catalytic activity, and facilitate membrane and cytoskeletal interactions.
SH3 domains thus represent targets for intervention in a number of pathologies since they occur in several critical intracellular signalling proteins.

### SUMMARY OF THE INVENTION

The present invention relates to a method for the screening of compounds of therapeutical value which possess the ability of inhibiting the interaction between a proline-rich peptide and a SH3 domain-comprising peptide, which compounds are able to selectively target proteins involved in a variety of intracellular signalling pathways.
In one general aspect, the present invention provides a method for the screening of a candidate compound for inhibiting the interaction between a proline-rich peptide and a SH3 domain-comprising peptide by Homogeneous Time-Resolved Fluorescence technique, said method comprising the steps of:

a) providing a buffer solution containing a proline-rich peptide which is labelled with a first fluorescent marker, wherein said first fluorescent marker is the first partner or the second partner of paired FRET fluorescence markers;
b) adding the candidate compound to the solution obtained at step a);
c) adding the SH3 domain-comprising peptide which is directly or indirectly labelled with a second fluorescent marker and wherein said second fluorescent marker is the second partner or the first partner of paired FRET fluorescence markers;
d) submitting the mixture obtained at step d) to a source of energy at a wavelength corresponding to the excitation wavelength of the first partner of paired FRET fluorescence markers and measuring the fluorescence signal at the emission wavelength of the second partner of paired FRET fluorescence markers;
e) comparing the fluorescence signal value obtained at step e) with the fluorescence signal value obtained when step b) is omitted to determine if the candidate compound inhibits the interaction between the proline-rich peptide and the SH3 domain-comprising peptide.

In a preferred embodiment the present invention provides a method for the screening of a candidate compound for inhibiting the interaction between a proline-rich peptide and a SH3 domain-comprising peptide by Homogeneous Time-Resolved Fluorescence technique, wherein the first fluorescent marker is the first partner and the second fluorescent marker is the second partner of paired FRET fluorescent markers.

In another preferred embodiment the present invention provides a method for the screening of a candidate compound for inhibiting the interaction between a proline-rich peptide and a SH3 domain-comprising peptide by Homogeneous Time-Resolved Fluorescence technique, wherein the first fluorescent marker is the second partner and the second fluorescent marker is the first partner of paired FRET fluorescent markers.

In a preferred method of screening of the invention the first partner of paired FRET fluorescent markers is Europium cryptate.

In another preferred method of screening of the invention the second partner of paired FRET fluorescent markers is XL665.

A general aspect of the invention provides a method of screening of the invention, wherein the SH3 domain-comprising peptide is indirectly labelled with a second fluorescent marker, in which case said SH3 domain-comprising peptide is covalently bound to a detectable molecule having specific affinity for a labelling reagent comprising the second fluorescent marker, said labelling reagent being added during step c), subsequently to the addition of the SH3 domain-comprising peptide.

In a preferred embodiment of the above method of screening the detectable molecule is a Glutathion S Tranferase (GST) protein.

In another preferred embodiment of the above method of screening the labelling reagent is an antibody directed to the GST protein which is labelled with the second fluorescent marker.

Another general aspect of the invention provides a method of screening of the invention wherein the proline-rich peptide is selected from the group consisting of the proline-rich peptides contained in the Fyn, Abl, CrkN, Src, Nsrc, PIK and Nef proteins.

A further general aspect of the invention provides a method of screening of the invention wherein the proline-rich peptide is selected from the group of proline-rich peptides contained in the p22phox, p47phox and p67phox sub-unit proteins of the human NADPH oxidase.

In a preferred embodiment of the method of screening of the invention, the proline-rich peptide is a proline-rich peptide contained in the p22phox, sub-unit protein of the human NADPH oxidase.

Another general aspect of the invention provides a method of screening, wherein the proline rich peptide comprises a proline rich amino acid sequence of approximately 10 amino acids in length which binds to an SH3 domain with a dissociation constant comprised between 5 and 100 μM.

In preferred embodiments of the method of screening of the invention, the proline rich peptide comprises a proline rich amino acid sequence selected from « Pro-X-X-Pro », « +-X-X-Pro-X-X-X-Pro », « Pro-X-X-Pro-X-+ », « +-X-q-Pro-X-q-Pro » and « q-Pro-X-q-Pro-X-+ », wherein X denotes any aminoacid residue, + denotes Arg or Lys and q denotes a hydrophobic aminoacid.

In other preferred embodiments of the method of screening of the invention, the proline rich peptide comprises a proline rich amino acid sequence selected from « R-X-#-Pro-X-X-Pro », « -Pro-X-X-Pro-X-R », « +-X-X-Pro-X-X-Pro», «Pro-X-X-Pro-X-+» , « Pro-X-@-X-X-Pro-X-X-Pro» and « Pro-X-X-D-Y», wherein X denotes any aminoacid residue, + denotes Arg or Lys, @ denotes an aromatic or aliphatic aminoacid residue and # denotes an aromatic aminoacid residue.

In a preferred embodiment of the invention the above method of screening is a method, wherein the proline-rich peptide comprises at least 10 consecutive proline rich amino acids from the amino acid sequence of SEQ ID N°3.

In a most preferred embodiment of the invention the above method of screening is a method, wherein, wherein the proline-rich peptide consists of the amino acid sequence of SEQ ID N°3.

The present invention also concerns a method of screening of the invention, wherein the proline-rich peptide has from 10 to 100 amino acids in length.

The present invention further concerns a method of screening of the invention, wherein the SH3 domain comprising peptide has from 55 to 100 amino acids in length.

In preferred embodiments of the method of screening of the invention, the SH3 domain of the SH3 domain-comprising peptide is selected from the group of SH3 domains contained in human Pl3K p85, mouse Abl, human Fyn, human c-Src, human Lck, C elegans SEM-5, mouse Grb2, human Grb2, mouse c-Crk, human 53BP2 and human Hck kinase proteins.

In other preferred embodiments of the method of screening of the invention, the SH3 domain of the SH3 domain-comprising peptide is selected from the group of SH3 domains contained in human PI3K p85, human Abl, human Fyn, human c-Src, human Lck, human Lyn, human Grb2, human PLC-γ, human 53BP2 human GAP, human 53BP2, human Csk and human Hck kinase proteins.

A further general aspect of the invention provides a method of screening wherein the SH3 domain of the SH3 domain-comprising peptide is selected from the group of SH3 domains contained in the p47phox and p67phox sub-unit proteins of the human NADPH oxidase.

In a preferred embodiment of the method of screening of the invention, the SH3 domain of the SH3 domain-comprising peptide is an SH3 domain contained in the p47phox sub-unit protein of the human NADPH oxidase.

In a most preferred embodiment of the method of screening of the invention, the SH3 domain-comprising peptide comprises the amino acid sequence of SEQ ID N°4.

The invention also concerns a screening reagent consisting of a proline-rich peptide which is labelled with a first fluorescent marker, wherein said first fluorescent marker is the first partner or the second partner of paired FRET fluorescence markers.

The invention further concerns a screening reagent of the invention, wherein the first fluorescent marker is a Europium cryptate molecule.

In a preferred embodiment the screening reagent of the invention consists of the proline-rich peptide having the amino acid sequence of SEQ ID N°3 which is covalently bound to a Europium cryptate molecule.

In another preferred embodiment the screening reagent of the invention consists of an SH3 domain-comprising peptide which is directly or indirectly labelled with a second fluorescent marker and wherein said second fluorescent marker is the second partner or the first partner of paired FRET fluorescence markers.

Another aspect of the invention is an above screening reagent, wherein the second fluorescent marker is XL665.

A further general aspect of the invention is a kit for the screening of a candidate compound for inhibiting the interaction between a proline-rich peptide and a SH3 domain-comprising peptide comprising:

a) a first screening reagent consisting of a proline-rich peptide which is labelled with a first fluorescence marker, wherein said first fluorescent marker is the first partner or the second partner of paired FRET fluorescence markers;
b) a second screening reagent consisting of a SH3 domain-comprising peptide which is directly or indirectly labelled with a second fluorescent marker and wherein said fluorescent marker is the second partner or the first partner of paired FRET fluorescence markers.

Another aspect of the invention is a screening reagent consisting of the SH3 domain-comprising peptide having the amino acid sequence SEQ ID N°4 which is fused to a GST protein.

Another general aspect of the invention is a complex formed between :

a) a proline-rich peptide which is labelled with a first fluorescent marker, wherein said first fluorescent marker is the first partner or the second partner of paired FRET fluorescence markers; and
b) a SH3 domain-comprising peptide which is directly or indirectly labelled with a second fluorescent marker and wherein said second fluorescent marker is the second partner or the first partner of paired FRET fluorescence markers.

A further general aspect of the invention is a complex formed between :

a) a proline-rich peptide which is labelled with a first fluorescent marker, wherein said first fluorescent marker is the first partner or the second partner of paired FRET fluorescence markers; and
b) a candidate compound which inhibits the interaction between the proline-rich peptide defined in a) and a SH3 domain-comprising peptide.

The invention also concerns a complex formed between :

a) a SH3 domain-comprising peptide which is directly or indirectly labelled with a second fluorescent marker and wherein said second fluorescent marker is the second partner or the first partner of paired FRET fluorescence markers; and
b) a candidate compound which inhibits the interaction between a proline-rich peptide and the SH3 domain-comprising peptide defined in a).

Another general aspect of the invention is the use of a candidate compound selected according to the method of screening of the invention for manufacturing a pharmaceutical composition.

## BRIEF DESCRIPTION OF THE DRAWINGS

**Fig.1**: schematic illustration of a specific embodiment of the screening method according to the invention useful for identifying compounds which inhibit the binding between the proline-rich region contained in the p22-*phox* protein and the SH3 domains contained in the p47-*phox* protein.

**Fig.2:** schematic illustration of a specific embodiment of the screening method according to the invention useful for identifying compounds which inhibit the binding between the proline-rich region contained in the p47-*phox* and the SH3 domain contained in the p67-*phox* protein.

**Fig.3:** Measures of the ΔF Ratio reflecting the binding between recombinant p47-*phox* $SH3_{AB}$ to recombinant p22-*phox*-EuK as a function of time (hours) for a final concentration of recombinant p47-*phox* $SH3_{AB}$ of 80nM.

**Fig.4:** ΔF Ratio measured for increasing final concentrations of recombinant p47-*phox* AB.

**Fig.5:** ΔF Ratio measures at increasing concentrations of recombinant p47-*phox* $SH3_{AB}$ for increasing amounts of anti-GST-XL 665. First curve (upper curve): anti-GST-XL665 at 800 ng/well. Second curve :anti-GST XL665 at 400 ng/well. Third curve: anti GST XL 665 at 200 ng/well. Fourth curve: anti GST XL 665 at 100 ng/well. Fifth curve (bottom curve) anti GST XL665 at 50 ng/well.

**Fig.6:** Comparison of the ΔF Ratio measures using either recombinant p47-*phox* $SH3_{AB}$ or recombinant mutated p47-*phox* $SH3_{AB}$. Upper curve : ΔF Ratio measures using wild type recombinant p47-*phox* $SH3_{AB}$. Bottom curve: ΔF ratio measures obtained using recombinant p47-*phox* $SH3_{AB}$ mutated by substitution of proline residue in position 156 by glutamine aminoacid residue.

**Fig.7:** Inhibition of the binding between p47-*phox* SH3 AB and p22-EuK peptide by unlabelled p22-EuK peptide.

**Fig.7A** illustrates the ΔF Ratio measures obtained by adding increasing final concentrations (nM) of unlabelled p22-EuK peptide to the assay.

**Fig. 7B** illustrates the IC50 calculation for the unlabelled p22-EuK peptide.

It is depicted the inhibition percentage of binding between p47-*phox* SH3 AB and p22-EuK peptides obtained with three different candidate compounds using either 96 wells assay (left blank bars) or the 384 wells high throughput assay using increasing amounts of p22-EuK peptide, respectively 0,4 ng/well (second bars), 0,8 ng/well (third bars) and 1,6 ng/well (fourth, i.e. right side bars).

**Fig. 8** illustrates the comparison between 96-well and 384-well format of inhibition percentage by 3 different candidate inhibitors in presence of increasing concentrations of p22-EuK peptide.

## DETAILED DESCRIPTION OF THE INVENTION

All SH3 domains have the same overall topology, consisting of two small β-sheets that are packed against each other approximately at right angles. There are three variable loops revealed when SH3 domains are compared, termed the RT, N-Src, and distal loops, this terminology, like the name of the domain itself, referring to features of the Src-tyrosine kinase. The peptide-binding surface of the SH3 domain consists of an elongated patch of aromatic residues, which are well-suited to binding relatively hydrophobic ligands such as proline-rich peptides . It has been shown that SH3 domains actually bind to proline-rich ligands which generally adopt the left-handed polyproline-ll helix (PPII) conformation, with two prolines that are on the same face of the helix and separated by two aminoacids being critical for binding and which may have the structural formula « Pro-X-X-Pro », wherein X is any aminoacid.

A typical example of interactions between SH3 domains and proline-rich peptides occur for stabilising the assembly of the different protein subunits of NADPH oxydase. The NADPH oxydase enzyme consists of at least four polypeptide components. Two of the components, termed gp91-*phox* and p22-*phox*, form an unusual heterodimeric cytochrome b, namely cytochrome $b_{558}$. The other two components, termed p47-*phox* and p67-*phox* are cytosolic proteins that assemble with cytochrome $b_{558}$ during activation of the enzyme.

At least one additional protein, the small GTP-binding protein Rac (either Rac1 or Rac2), is needed for NADPH oxydase activation. A sixth oxydase-related protein, termed p40-*phox,* has been recently identified and has sequence similarity to p47-*phox* and p67-*phox* and appears to physically associate with p67-*phox.* Defects in any of the four genes that code for components of the NADPH oxydase enzyme system can cause pathologies such as chronic granulomatous diseases. Each of p47-*phox* and p67-*phox* contain two SH3 domains. The newly described p40-*phox* also contains one SH3 domain. The SH3 domains of both p67-*phox* and p47-*phox* are necessary for NADPH oxydase activation, since deletion mutants of p67-*phox* lacking one or both SH3 domains are unable to restore the ability to activate NADPH oxydase when transfected into p67-*phox* deficient B-cell lines derived from patients with this form of CGD ( DE MENDEZ et al., 1994). Interactions between proline-rich sequences in NADPH oxydase components and SH3 domains of p47-*phox* and p67-*phox* have recently been identified. The first is binding between the second SH3 domain of p67-*phox* and the COOH-terminal proline-rich sequence in p47-*phox.* The second interaction involves binding of the SH3 domains of p47-*phox* to a cytoplasmic region of p22-*phox* containing three proline-rich sequences. A natural mutation in the middle proline-rich region in p22-*phox* has been reported in a patient with CGD. This mutation was introduced into GST-fusion proteins and resulted in markedly reduced binding of GST-p47-SH3 (LETO et al, 1994). This group showed that synthetic peptides containing the middle proline-rich sequence of p22-*phox* (aa 149-162) abolished the binding between GST-p47-SH3 and GST-p22 (aa 127-195), while peptides based on the COOH-terminal proline-rich sequence (aa 170-195) or containing the p22-*phox* mutation P156Q were ineffective. These results suggest that the interaction between the SH3 domains of p47-*phox* and p22-*phox* depends on Pro156 and this results strongly suggests that this proline-dependent interaction is critical for assembly of an active enzyme.

It flows from the preceding analysis of prior art that several assays for detecting or assessing interactions between several SH3 domains and proline-rich ligands have been designed.

For example, LETO et al. (1994), have carried out binding assays for assessing interaction between NADPH oxydase p47-*phox* and p22-*phox.* For this purpose, GST-SH3 fusion proteins were biotinylated before being used to probe nitro-cellulose blots of proteins previously blocked in 5% non-fat milk. Detection of interactions were performed with anti-p67-*phox* antibodies by immunoblotting. In this assay, both full length p47-*phox* and p22-*phox* were used. Similar techniques of immunoblotting were carried out for assessing the specificity of p47-*phox* SH3-domain interactions in NADPH oxydase assembly and activation DE MENDEZ I. et al., (1997).

The assembly and activation of the phagocyte NADPH oxydase has also been studied by SUMIMOTO et al. (1996). These authors have assessed interactions between p47-*phox* and p22-*phox* using either full length or partial aminoacid sequences of these proteins under the form of fusion proteins with GST. The assay for binding of SH3 domains of p47-*phox* to the C-terminal cytoplasmic tail of p22-*phox* included transfer of purified GST fusion proteins containing various regions of p22-*phox* to nitro-cellulose membrane subsequently blocked by buffer and then incubated with GST-fusion proteins with various SH3 domains of p47-*phox,* the filter being then probed with an anti-GST monoclonal antibody in order to detect the presence of an interaction. These authors had also performed two-hybrid experiments in yeast by using a first vector containing the C-terminal cytoplasmic domain of p22-*phox* and a second vector containing the tandem SH3 domains of p47-*phox*, the presence of an interaction between the two NADPH oxydase sub-unit proteins being revealed by a β-galactosidase assay.

Additionally, these authors had designed a real-time interaction assay of SH3 domains with p22-*phox* which included an immobilisation of GST-p22-*phox* and detection of interaction of the thus immobilised protein with an analysis through laser biosensor measures of real-time optical changes.

The different assays detailed above are satisfactory for assessing the presence of absence of binding between a first protein containing one or several SH3 domains and a second protein containing a proline-rich region.

Taking into account the importance of the interactions between SH3 domains and proline-rich regions of proteins involved in intracellular pathway signalling, as well as the involvement of proteins bearing such peptide motifs in the occurrence of various diseases, the inventors have now designed a specific assay for screening compounds which possess the ability of inhibiting the interaction between a proline-rich peptide and an SH3 domain comprising peptide, such a compound enabling a regulation or a modulation of the biological activity of various intracellular signalling pathway proteins for preventing or curing the diseases associated with transcription, translation or biological activity regulation defects of such proteins.

In order to design such a screening method for those inhibitor compounds, the inventors had to avoid several drawbacks of the available prior art assays, since such a screening method will need to satisfy various requirements, such as:

a) specificity of the binding interactions detected in the assay;
b) sensitivity of the assay, even in conditions wherein a high level of release of one of the binding partner proteins is observed;
c) use of easily synthetized reagents;
d) reproducibility of the method;
e) enablement of a high throughput scanning of candidate inhibitor compounds.

These necessary requirements for a screening method for candidate inhibitor compounds are not fulfilled by the prior art techniques detailed above.

The immunoblotting techniques involve the immobilisation of the first partner protein on a membrane, usually a nitrocellulose membrane, which may significantly influences the spatial conformation of said immobilised protein and may substantially alter its subsequent interactions with the second partner protein, either by decreasing or increasing its affinity as regards the affinity which would be observed during the naturally occurring binding of these two partner proteins.

Moreover, several prior art assays made use of two partner proteins both under the form of GST fusion proteins, which cause an enhancement in non specific interactions between the two partner proteins, since it is well known in the art that the GST protein easily dimerizes and may thus artefactually stabilise weak or non-specific interactions between the two partner proteins.

There is no need to say that the non-specific interactions occurring in immunoblotting assays do not alters solely specificity of the interaction which is sought, but also the sensitivity and the reproducibility of the assay. Further, immunoblotting assays require the use of large amounts of each of the partner proteins which are not suitable for performing large scale screening methods.

Additionally, these methods are not suited for high throughput screening.

Real-time interaction analysis of the binding of two partner proteins also involve the immobilisation of at least one of said partner proteins which does not allow the testing of binding events in environmental conditions as close as possible to the binding that naturally occurs *in vivo*.

The inventors have now found a method which involves specific binding interactions between a proline-rich peptide and a SH3 domain-comprising peptide, the specificity, sensitivity and reproducibility of which allow its use for screening compounds that are able to inhibit this specific interaction which occurs in an homogeneous liquid phase without immobilisation of anyone of the interacting protein partners.

Further, the inventors have found that the screening method of the invention does not require the use of full length proteins nor of large polypeptides because there is no need for aminoacid sequences flanking the interacting SH3 domains and proline-rich motifs that are used to stabilise binding in immunoblotting assays or alternatively that are used as linking arms bound to the reaction cuvette in real-time interaction analysis assays.

It was unexpected that the binding of such a small proline rich sequence to a SH3 domain comprising peptide could be visualized.

Furthermore, the interaction between the SH3 domain comprising peptide and such a small proline-rich peptide being relatively weak (ranging from 5 to 100 μM) it was unexpected that this interaction could be tested in HTRF.

Thus, the invention firstly concerns a method for the screening of a candidate compound for inhibiting the interaction between a proline-rich peptide and a SH3 domain-comprising peptide by Homogeneous Time-Resolved Fluorescence technique, said method comprising the steps of:

a) providing a buffer solution containing a proline-rich peptide which is labelled with a first fluorescent marker, wherein said first fluorescent marker is the first partner or the second partner of paired FRET fluorescence markers;
b) adding the candidate compound to the solution obtained at step a);
c) adding the SH3 domain-comprising peptide which is directly or indirectly labelled with a second fluorescent marker and wherein said second fluorescent marker is the second partner or the first partner of paired FRET fluorescence markers;
d) submitting the mixture obtained at step d) to a source of energy at a wavelength corresponding to the excitation wavelength of the first partner of paired FRET fluorescence markers and measuring the fluorescence signal at the emission wavelength of the second partner of paired FRET fluorescence markers;
e) comparing the fluorescence signal value obtained at step e) with the fluorescence signal value obtained when step b) is omitted to determine if the candidate compound inhibits the interaction between the proline-rich peptide and the SH3 domain-comprising peptide.

Homogeneous time resolved fluorescence technique consists of an homogeneous assay wherein every component is completely in solution and which combines the benefits of homogeneous assay formats, the robustness and sensitivity of radio-isotopic method and the safety and stability of non-isotopic, fluorescent labels. The HTRF technique makes use of the fluorescence resonance energy transfer, which is a non-radioactive process whereby energy from a fluorescent donor molecule is transferred to a fluorescence acceptor without the involvement of a photon (FÖRSTER, 1948, 1949). One result of this interaction is that excitation of the donor molecule enhances the fluorescence emission of the longer wavelength acceptor molecule (i.e. sensitised acceptor emission).

The quantum yield of the donor fluorescent emission is concomitantly diminished. The efficiency of energy transfer has a strong inverse dependence on the distance between the donor and acceptor molecules. Thus, FRET is a highly specific indicator of the proximity of the two molecules.

Depending on the nature of the respective donor and acceptor fluorescent molecules, the distance range over which resonance energy transfer can occur may vary. For several paired FRET fluorescence markers, it is widely accepted that resonance energy transfer occurs within a distance range of 10-100 Angstroms between the donor and the acceptor molecules. For other paired FRET markers, such as Eucryptase and XL665, it is predicted that 50% energy transfer occurs at a distance of 9 nm and a 75% energy transfer occurs at 7.5 nm.

According to the method of the invention, the presence of a binding interaction between the proline-rich peptide and the SH3 domain comprising peptide is optically detected by the measure of the fluorescence signal emitted at the emission wavelength of the acceptor FRET fluorescence marker, the level of the fluorescence signal reflecting the affinity or the number of interactions occurring between the proline-rich peptide and the SH3 domain comprising peptide in the homogeneous assay in solution. Indeed, the level of the fluorescence signal measured directly reflects the equilibrium between the number of bound and the number of free protein partners in solution and allow to measure the real affinity of the binding interaction between these proteins. Additionally, the displacement of the binding equilibrium by a candidate molecule that inhibit the interaction between the proline-rich peptide and the SH3 domain comprising peptide is also directly measured by the evaluation of the decrease in the fluorescence signal at the acceptor molecule emission wavelenqth and thus enables to select. among the candidate compounds to be assayed, those for which a high binding equilibrium displacement is measured.

The term « peptide » is intended to designate a polymeric compound comprised of covalently linked aminoacid residues having an aminoacid length from 10 to 100 aminoacids.

A proline-rich « peptide » is intended to have an aminoacid length from 10 to 100 aminoacids.

For the purpose of the present invention, a proline-rich peptide » comprises a proline-rich aminoacid sequence motif that possess high affinity binding properties to a SH3 domain and thus consists of a SH3-binding site. The proline-rich aminoacid sequence motif contained in a proline-rich peptide of the invention has approximately 10 aminoacids in length and bind to a SH3 domain with dissociation constant comprised between 5 and 100 μM. In the method of screening of the invention, the proline rich peptide has to bind to an SH3 comprising peptide with a dissociation constant below 10μM, preferably below 5μM, more preferably below 1μM.

The one skilled in the art may advantageously refer to various published articles for an exhaustive structural and functional definition of a proline-rich aminoacid sequence motif, such as the articles of PAWSON (1995), REN R. et al. 1993, mouse mSos, YU et al. (1994 PI3Kp85, human), CHEN et al. (1993), FENG et al. (1994), LIM et al. (1994), MUSACCHIO A. et al. (1994, 3BP-1, human), WITTEKIND et al. (1994), RICKLES et al. (1994), CHERNIACK et al. (1994), MAYER BJ, Cesareni et al. (2002) and ECK MJ. Each of these articles being herein incorporated by reference.

Typical proline-rich aminoacid sequence motifs are respectively « Pro-X-X-Pro », « +-X-X-Pro-X-X-X-Pro », « Pro-X-X-Pro-X-+ », « +-X-q-Pro-X-q-Pro » and « q-Pro-X-q-Pro-X-+ », wherein X denotes any aminoacid residue, + denotes Arg or Lys and q denotes a hydrophobic aminoacid.

More specifically, typical proline-rich aminoacid sequence motifs are respectively « Pro-X-X-Pro », « Arg-X-X-Pro-X-X-X-Pro », « Pro-X-X-Pro-X-Arg », « Arg-X-q-Pro-X-q-Pro » and « q-Pro-X-q-Pro-X-Arg »,
wherein X denotes any aminoacid residue and q denotes a hydrophobic aminoacid.

Other typical proline-rich aminoacid sequence motifs are respectively « R-X-#-Pro-X-X-Pro », « -Pro-X-X-Pro-X-R », « +-X-X-Pro-X-X-Pro», «Pro-X-X-Pro-X-+» , « Pro-X-@-X-X-Pro-X-X-Pro» and « Pro-X-X-D-Y», wherein X denotes any aminoacid residue, + denotes Arg or Lys, @ denotes an aromatic or aliphatic aminoacid residue and # denotes an aromatic aminoacid residue.

For instance, the proline-rich aminoacid sequence motif contained in the p22-*phox* sub-unit protein of the NADPH oxidase has the following aminoacid sequence: « PPSNPPPRPP ».

A proline-rich peptide according to the invention include peptides containing the proline-rich aminoacid sequence motifs comprised in the Fyn, Abl, CrkN, GrbN, Src, Nsrc and PIK proteins that are disclosed in the article of MAYER and ECK (1995).

A proline-rich peptide according to the invention also includes peptides containing the proline-rich aminoacid sequence motifs comprised in the Nef proteins from HIV (disclosed in LIM et al., 1996) which bind to the SH3 domains of Lck, Hck, Lyn and Fyn.

For the purpose of the present invention, a « SH3 domain-comprising peptide » consists of a peptide containing one, two or three aminoacid sequence motifs of a SH3 domain, which SH3 domain possess a high binding affinity with a proline-rich aminoacid sequence motif such as defined above. The SH3 (Src homology 3) domain is a conserved aminoacid sequence motif of 55 to 70 aminoacids in length that recognises proline-rich ligands in target protein, thereby mediating protein-protein intracellular interactions.

A SH3 domain is not endowed with conserved aminoacid sequence and is thus primarily defined functionally as an aminoacid sequence motif that binds with a high affinity to a proline-rich aminoacid sequence motif.

For the structural features of various SH3 domain aminoacid sequence motifs, the one skilled in the art may advantageously refer to the articles of PAWSON T. (1995), AGHAZADEAH et al. (1999), DALGARNO et al. (1998), MUSACCHIO et al. (1992; Spectrin SH3, chicken), YU H. et al. (1992; c-Src SH3, chicken), (KOYAMA et al. (1993); PI3K p85 SH3, human), BOOKER et al. (1993; PI3K p85 SH3, bovine), NOBLE et al. (1993; Fyn SH3, human), KOHDA et al. (1993; PLC-y SH3, human), BORCHERT et al. (1994; Csk SH3, human), KOHDA et al. (1994; Grb2 SH3, human), YANG et al. (1994; GAP SH3, human), GOSSER et al. (1995; Abl SH3, human), ZHANG et al. (1995; Drk SH3, Drosophila), GRUPRASAD et al. (1995; Grb2, human), MORTON et al. (1996; Fyn SH3, human), MAIGNAN et al. (1995; Grb2 SH3-SH2-SH3, human), BLANCO et al. (1997; Spectrin SH3, chicken), HIROAKI et al. (1996; Lck SH3, human), NAM et al. (1996; Abl SH2-SH3, human), LIANG et al. (1996; PI3K p85 SH3, human), KISHAN et al. (1997; Eps 8 SH3, mouse), LIM et al. (1994; SEM-5, C. *Elegans),* WITTEKIND et al. (1994; Grb2, mouse), KEFALAS et al. (1995; Lyn) and GORINA et al. (1996; 53BP2, human), these articles being herein incorporated by reference.

The screening method of the invention may also be carried out with a SH3 domain-comprising peptide selected from the group consisting of SH3 domains contained in human PI3K p85, mouse Abl, human Fyn, human c-Src, human Lck, C.elegans SEM-5, mouse Grb2, human Grb2, mouse c-Crk, human 53BP2 and human Hck kinase proteins.

More preferably the screening method of the invention may be carried out with a SH3 domain-comprising peptide selected from the group consisting of SH3 domains contained in human PI3K p85, human Abl, human Fyn, human c-Src, human Lck, human Lyn, human Grb2, human PLC-γ, human 53BP2 human GAP, human 53BP2, human Csk and human Hck kinase proteins.

The proline-rich peptide and the SH3 domain-comprising peptide may be obtained by chemical synthesis, such as described by MERRIFIELD et al. (1965a; 1965b). These peptides may also be obtained by genetic engineering methods, such as described in SAMBROOK et al. (1989).

According to the screening method of the invention, each of these interacting peptides, namely the proline-rich peptide and the SH3 domain-comprising peptide is labelled with one fluorescent marker consisting in the donor molecule or the first partner of paired FRET fluorescence markers or in the acceptor molecule or the second partner of paired FRET fluorescence markers.

Indeed, when the first fluorescent marker labelling the proline-rich peptide is the acceptor molecule or the first partner of paired FRET fluorescence markers, the second fluorescent marker labelling the SH3 domain-comprising peptide is the acceptor molecule or the second partner of the paired FRET fluorescence markers.

Conversely, when the first fluorescent marker labelling the proline-rich peptide is the acceptor molecule or the second partner of paired FRET fluorescence markers, the second fluorescent marker labelling the SH3 domain-comprising peptide is the acceptor molecule or the first partner of the paired FRET fluorescence markers used.

In a most preferred embodiment, the first fluorescent marker labelling the proline-rich peptide is covalently linked to said proline-rich peptide, advantageously at the C-terminal and/or the N-terminal end of the proline-rich peptide.

The SH3 domain-comprising peptide may be either directly or indirectly labelled with the second fluorescent marker.

In the specific embodiment wherein the SH3 domain-comprising peptide is directly labelled with the second fluorescent marker, said second fluorescent marker is most preferably covalently linked to the SH3 domain-comprising peptide, most preferably at either the N-terminal end or the C-terminal end of the SH3 domain comprising peptide.

In the specific embodiment wherein the SH3 domain-comprising peptide is indirectly labelled with the second fluorescent marker, said SH3 domain-comprising peptide is covalently bound to a detectable molecule which has specific affinity for a labelling reagent comprising the second fluorescent marker.

According to this specific embodiment, the detectable molecule may consist of a polypeptide which is able to be recognized by the labelling reagent. Most preferably, the detectable molecule is covalently bound at the N-terminal end or the C-terminal end of the SH3 domain-comprising peptide.

According to this specific embodiment, the labelling reagent comprises preferably an antibody which recognizes specifically the detectable molecule which is covalently bound to the SH3 domain-comprising peptide. The term « antibody », as used herein, encompasses polyclonal and monoclonal antibody as well as the antigen recognizing domains of an antibody such as Fab, $F(ab)'_2$ as well as single chain Fv antibody and a chimeric antibody such as a humanised antibody. The labelling reagent may also comprise other ligands which have affinity for the detectable molecule that is covalently bound to the SH3 domain-comprising peptide, such as streptavidin, in which case the detectable molecule is advantageously a biotin protein.

The labelling reagent also comprises the second fluorescent marker which is preferably covalently bound to the ligand that possesses affinity for the detectable molecule bound to the SH3 domain-comprising peptide.

According to this specific embodiment wherein the SH3 domain-comprising peptide is indirectly labelled with the second fluorescent marker, as detailed above, said labelling reagent is added during step c) of the method of the invention, subsequently to the addition of the SH3 domain-comprising peptide.

In a most preferred embodiment of the screening method of the invention, the proline-rich peptide is labelled with a first fluorescent marker which consists of the donor fluorescent marker or the first partner of paired FRET fluorescence markers; the SH3 domain comprising peptide is covalently bound to a detectable molecule, preferably a protein; the labelling reagent comprises an antibody directed against said detectable protein which is covalently bound to the second fluorescent marker which is the acceptor fluorescent marker or the second partner of the paired FRET fluorescence markers.

Every paired FRET fluorescence markers known in the art may be used for performing the screening method of the invention.

However, in a preferred embodiment of said screening method, the donor fluorescent marker or the first partner of paired FRET fluorescent markers consists of Europium cryptate, in which case the acceptor fluorescent marker or the second partner of the paired FRET fluorescent markers is XL665.

Rare earth cryptates are disclosed by and have the necessary properties for enhancing Europium fluorescence, since they act as an energy antenna for collecting the excitation energy and transferring it to Europium, protects the Europium from quenching, does not interfere with the fluorescent emission and can be used to conjugate the Europium to biological macromolecules. Europium cryptate has a molecular weight of approximately 1kDa. With Europium cryptate as the donor fluorescent molecule or the first partner of paired FRET fluorescence markers, the acceptor fluorescent marker of the second partner of the paired FRET fluorescent markers is most preferably a modified allophycocyanine which is termed XL665 and has a molecular weight of approximately 105 kDa. XL665 notably has the necessary characteristics of an acceptor, XL665 also has the additional advantage of producing a signal amplification by energy transfer. This is due in part to the high quantum yield (0.7) of XL665.

In the specific embodiment wherein the paired FRET fluorescent markers are respectively Europium cryptate and XL665, the mixture obtained at step c) of the method according to the invention is submitted to a source of energy at a wavelength of 337 nm, which is the excitation wavelength of Europium cryptate and the fluorescence signal is measured at 665nm, which is the emission wavelength of XL665.

The candidate inhibitor compound to be assayed by carrying out the method of the invention may be of any nature, being either an organic, mineral or biological molecule.

In all cases, the ability of the candidate compound to inhibit the binding interactions between the proline-rich peptide and the SH3 domain-comprising peptide may be calculated as it follows:

a) **Ratio**

It is first calculated the ratio (R) between the fluorescence signal obtained at the emission wavelength of the acceptor fluorescent marker or second partner of the paired FRET fluorescence markers and the fluorescence signal at the emission wavelength of the donor fluorescent marker or the second partner of the paired FRET fluorescent marker, which is expressed by the following formula:

Ratio(R) = (emission fluorescence signal of second partner/emission fluorescence signal of first partner) x 1000.

**b) ΔRatio**

The Δ Ratio value represents the difference between the value of the sample ratio (R) and the negative control ratio (R').

The sample ratio (R) value is obtained when the assay conditions include incubation of (i) the proline-rich peptide labelled with the first fluorescent marker and (ii) the SH3 domain-comprising peptide which is directly or indirectly labelled with the second fluorescent marker.

The negative control ratio (R') is obtained in assay conditions including incubation of (i) the proline rich peptide which is labelled with the first fluorescent marker and (ii) the second fluorescent marker alone or the labelling reagent comprising said second fluorescent marker.

The Δ Ratio value is calculated according to the following formula:

ΔRatio (ΔR)= sample ratio (R) - negative control ratio (R').

The value of the Δ ratio represents the signal specific of the assay and is dependent of the apparatus used for carrying out the method of the invention.

**c) ΔF value**

This value represents the specific signal minus the background signal and is independent of the apparatus used for carrying out the method of the invention. The ΔF value is expressed according to the following formula:

ΔF = (Δratio(R)/negative control ratio (R')) x 100.

The ΔF value is expressed as a percentage (%).

As used herein, the fluorescence signal referred to in the general definition of the screening method according to the invention encompasses the ΔF (%) value.

An illustrative example of the computerisation of the ΔF value obtained with a proline-rich peptide derived from p22-*phox* protein labelled with Europium cryptate and an SH3 domain-comprising peptide derived from the p47-*phox* protein indirectly labelled with XL665 is shown in the examples below.

Further, the measures above are performed with increasing amounts of a candidate inhibitor compound to be assayed as well as without said candidate inhibitor compound and the IC50 value for this specific candidate compound is determined by plotting the ΔF value obtained (ordinates) against the final concentration of the candidate compound being assayed in the assay (abscissa). The IC50 value is determined as the final concentration of the candidate inhibitor compound for which 50% inhibition of the ΔF value is observed, as compared with the ΔF value which was obtained in the absence of the candidate inhibitor compound in the assay.

The high specificity of the screening method according to the invention has been shown, since a single aminoacid substitution in the SH3 domain-comprising peptide aminoacid sequence totally abolishes the binding interactions between the mutated peptide and the corresponding proline-rich peptide.

The high sensitivity of the assay has also been demonstrated, as shown in the examples below, for instance by using an unlabelled proline-rich peptide for competing with the proline-rich peptide labelled with the first fluorescent marker for binding to the SH3 domain-comprising peptide.

It has also been shown that the method according to the invention was efficient in detecting various organic compounds that were able to inhibit the binding interactions between the proline-rich peptide and the SH3 domain-comprising peptide, such as various coumarine and quinazoline compounds, specifically in the embodiment wherein the proline-rich peptide is derived from the p22-*phox* protein and the SH3 domain comprising peptide is derived from the p47-*phox* protein, both proteins being subunits of the NADPH oxidase complex.

Moreover, the inventors have shown a high correlation between the ability of a given compound to inhibit the binding between the proline-rich peptide derived from the p22-*phox* protein and the SH3 domain-comprising peptide derived from the p47-*phox* protein with the ability of the same inhibitor compound to inhibit NADPH oxidase activity, as measured either in a cell free assay or in a secondary HL60 cell based assay.

These results clearly demonstrate the biological significancy of the data obtained by carrying out the screening method of the invention and its relevance for screening compounds which will exert their inhibitory properties in *in vivo* conditions.

As already mentioned, defects in the regulation of signalling pathway proteins are involved in various pathologies and there is thus a need for identifying biologically active inhibitor compounds susceptible to therapeutically act for preventing or curing such diseases. The method of the invention as defined above will no allow to select compounds of great therapeutical value which are able to inhibit interaction between proline-rich reagent and SH3 domain contained in these signalling pathway proteins.

Consequently, the invention encompasses the above screening method wherein the SH3 domain-comprising peptide is derived from a signalling protein the deregulation of which is associated with the development of a given pathology.

An illustrative but not limiting examples of proteins from which the SH3 domain-comprising peptide may be derived include the following proteins: Lyn (SAKSELA et al., 1995; AIDS), Hck (SAKSELA et al., 1995; AIDS), Lyn (YAMACHITA et al., 1994; Allergy and Asthma), Grb2 (JAMES et al., 1994; (DALY et al., 1994; Breast Cancer ), Src (LUTTRELL et al, 1994; Breast Cancer), p85 (JHUN et al., 1994);( HU et al..Cancer), Grb2 (RAVICHNDRAN et al., 1995; GOTTESMANN, 1994; CHARDIN et al. , 1993; LI et al., 1993; Cancer), Gap (MOODIE et al., 1994; Cancer), Grb2(PENDERGAST et al. 1993; CML and ALL), CrkL (ODA et al., 1994; CML and ALL), STATs (IHLE, 1994; DARNELL et al., 1994; Inflammatory disease), p47-*phox* and p67-*phox* (LETO et al., 1994; Inflammatory disease), Btk (DE WEERSE et al., 1994; Pre-B-Cell Leukemia), Tec (SATO et al., 1994; myelodysplastic syndrome ), Src (KAPLAN et al., 1994; LOWE et al., 1993; osteoporosis ), each of the above articles being incorporated herein by reference.

In a typical embodiment of the screening method of the invention, the proline-rich peptide and the SH3 domain comprising peptide are derived from subunit proteins belonging to the NADPH oxidase complex. The NADPH oxidase, which is predominantly expressed in polymorphonuclear leukocytes or neutrophils, is activated during the stimulation of neutrophils during an immune response to foreign pathogens and produce superoxyde anion ($O_2^-$). $O_2^-$ is then rapidly converted to secondary toxic oxygen species such as hydrogen peroxyde ($H_2O_2$), hydroxyl radical (OH.) and hypochlorous acid (HOCl). Although the objective of this process is to remove infectious agents, foreign particles and damage tissues from the body, neutrophil-generated reactive oxygen species can also damage host tissues in the vicinity of the inflammatory site. Indeed, reactive oxygen species have been reported to be involved in the tissue injury associated with a number of inflammatory diseases, including rheumatoid arthritis, ischemia-reperfusion injury and acute respiratory distress syndrome.

It flows from the preceding experimental data that the finding of the compounds having the ability to inhibit the binding between SH3 domains and proline-rich region of the different protein subunits of the NADPH oxidase complex have therapeutical value for preventing or curing inflammation situations, COPD, atherosclerosis, ARDS, septic shock, as well as general aging processes and cancer.

Useful inhibitor compounds for inhibiting NADPH oxydase activity encompass the following compounds :

(i) compounds which inhibit the interaction between the proline-rich region contained in the p22-*phox* protein and the first SH3 domain contained in the p47-*phox* protein;

(ii) compounds inhibiting the interaction between the proline-rich region contained in the C-terminal end of the p47-*phox* protein and the SH3 domain contained in the N-terminal end of the p67-*phox* protein; and

(iii) compounds that inhibit the binding interactions between the proline-rich region contained in the p67-*phox* protein and the second SH3 domain contained in the p47-*phox* protein.

An illustration of a specific embodiment of a screening method according to the invention useful for identifying compounds which inhibit the binding between the proline-rich region contained in the p22-*phox* protein and the SH3 domains contained in the p47-*phox* protein is shown on figure 1.

According to this specific embodiment of the method of the invention, a proline-rich peptide of 27 aminoacids in length derived from the p22-*phox* protein is labelled with a first fluorescent marker consisting of Europium cryptate. The SH3 domain-comprising peptide consists of a peptide containing the two SH3 domains derived from the p47-*phox* proteins which has 134 aminoacids in length and which peptide is coupled with a detectable molecule consisting of a Glutathion S transferase (GST). The SH3 domain comprising peptide is indirectly labelled with the second fluorescent marker through the labelling reagent which consists of an anti-GST antibody coupled to the second fluorescent marker which is XL665.

According to this embodiment, step d) of the method according to the invention is performed by submitting the homogeneous solution mixture containing the proline-rich peptide and the indirectly labelled SH3 domain comprising peptide to a source of energy at a wavelength of 337 nm, which is the excitation wavelength of Europium cryptate, and measuring the fluorescence signal both at 665 nm, which is the emission wavelength of the XL665, and at 620 nm, which is the emission wavelength of Europium cryptate. The assay is performed both in the absence of any candidate inhibitor compound and in the presence of increasing final concentrations of a given inhibitor candidate compound and computerization of the IC50 value is performed as detailed above.

Another illustration of a specific embodiment of the screening method according to the invention is depicted in figure 2, said illustrated embodiment being useful for selecting compounds that inhibit the binding interaction between the proline-rich region contained in the p47-*phox* protein and the C-terminal SH3 domain contained in the p67-*phox* protein.

Thus, in a preferred embodiment for the screening method of the invention, the proline-rich peptide is selected from the group of proline-rich peptides contained in the p22-*phox,* p47-*phox* and p67-*phox* subunit proteins of the human NADPH oxidase.

Most preferably, the proline-rich peptide comprises at least ten consecutive aminoacids from the aminoacid sequence of SEQ ID N°3.

Alternatively, the proline-rich peptide consists in the aminoacid sequence of SEQ ID N°3, which is encoded by the nucleic acid sequence SEQ ID N°1.

In another preferred embodiment of the screening method according to the invention, the SH3 domain-comprising peptide is selected from the group of SH3 domains contained in the p47-*phox* and p67-*phox* subunit proteins of the human NADPH oxidase.

In a most preferred embodiment, the SH3 domain comprising peptide comprises the aminoacid sequence of SEQ ID N°4 which is encoded by the nucleic acid sequence SEQ ID N°2.

Alternatively, the SH3 domain comprising peptide consists of the aminoacid sequence of SEQ ID N°4.

In another aspect, the present invention also concerns a screening reagent consisting of a proline-rich peptide which is labelled with a first fluorescence marker, wherein said first fluorescence marker is the first partner or the second partner of paired FRET fluorescence markers.

In a first preferred embodiment, the first fluorescent marker contained in the screening reagent above is a Europium cryptate molecule.

In a second preferred embodiment, the screening reagent consists of the proline-rich peptide having the aminoacid sequence of SEQ ID N°1 which is covalently bound to a Europium cryptate molecule.

In a further aspect, the invention also relates to a screening reagent consisting of a SH3 domain-comprising peptide which is directly or indirectly labelled with a second fluorescent marker and wherein said second fluorescent marker is the second partner or the first partner of paired FRET fluorescence markers.

In a first preferred embodiment of the screening reagent above, the second fluorescent marker is XL665.

In a second preferred embodiment, the screening reagent consists of the SH3 domain comprising peptide having the aminoacid sequence SEQ ID N°2 which is fused to a GST protein. In this second preferred embodiment, wherein the SH3 domain comprising peptide is indirectly labelled with the second fluorescent marker, the indirect label consists of a labelling reagent which is composed of an antibody directed against the GST protein which is covalently bound to the second fluorescent marker XL665.

The invention also deals with a kit for the screening of a candidate compound for inhibiting the interaction between a proline-rich peptide and a SH3 domain-comprising peptide comprising:

a) a first screening reagent consisting of a proline-rich peptide which is labelled with a first fluorescence marker, wherein said first fluorescent marker is the first partner or the second partner of paired FRET fluorescence markers;
b) a second screening reagent consisting of a SH3 domain-comprising peptide which is directly or indirectly labelled with a second fluorescent marker and wherein said fluorescent marker is the second partner or the first partner of paired FRET fluorescence markers.

In a further aspect, the invention deals with a complex formed between:

a) a proline-rich peptide which is labelled with a first fluorescent marker, wherein said first fluorescent marker is the first partner or the second partner of paired FRET fluorescence markers; and
b) a SH3 domain-comprising peptide which is directly or indirectly labelled with a second fluorescent marker and wherein said second fluorescent marker is the second partner or the first partner of paired FRET fluorescence markers;

The invention also concerns a complex formed between:

a) a proline-rich peptide which is labelled with a first fluorescent marker, wherein said first fluorescent marker is the first partner or the second partner of paired FRET fluorescence markers; and
b) a candidate compound which inhibits the interaction between the proline-rich peptide defined in a) and a SH3 domain-comprising peptide.

Another object of the invention consists of a complex formed between:

a) a SH3 domain-comprising peptide which is directly or indirectly labelled with a second fluorescent marker and wherein said second fluorescent marker is the second partner or the first partner of paired FRET fluorescence markers; and
b) a candidate compound which inhibits the interaction between a proline-rich peptide and the SH3 domain-comprising peptide defined in a).

The present invention also pertains to the use of a candidate compound which has been selected according to the method of the invention for manufacturing a pharmaceutical composition.
The present invention is further illustrated, without being limited, to the following figures and examples below.

## EXAMPLES

### Example 1 : Production of the SH3 domain-comprising peptide GST-p47*phox* $SH3_{AB}$.

### 1.1 Engineering of Glutathione S-Transferase Fusion p47*phox* $SH3_{AB}$ expression vector

The DNA fragment (SEQ ID N° 2) encoding amino acid residues 151-284 of p47*phox* (SEQ ID N° 4) was amplified by the polymerase chain reaction (PCR), using primers incorporating *Bam*HI and *Eco*RI sites at their 5' and 3'ends, respectively.
The amplified product was cloned between *Bam*HI and *Eco*RI restriction sites of the *Escherichia coli* expression vector pGEX-2T (Pharmacia Biotech Inc.), to produce the plasmid pGEX-2T-p47*phox*$SH3_{AB}$.
Construction was verified by nucleotide sequencing.

### 1.2 Production of the GST-p47*phox* $SH3_{AB}$.recombinant fusion protein

The plasmid encoding the GST-fusion protein was introduced into *E. coli*-strain DH5α.
Transformants were cultured overnight at 37°C in LB medium supplemented with ampicillin at 50 µg/ml. Overnight culture was diluted 1:100 into fresh 1L-LB medium and grown at 30°C to an OD of 0.8. Expression of the fusion protein was then induced at 30°C by addition of isopropyl-1-thio-β-D-galactopyranoside to 0.4 mM.
After 1 h, bacteria were centrifuged for 15 minutes at 2000g, +4°C and frozen.

### 1.3 Purification of the GST-p47*phox* $SH3_{AB}$ recombinant fusion protein

#### 1.3.1 Preparation of bacterial lysate

The pellet from the 1L-bacterial culture was then resuspended in 15ml of ice-cold PBS 1X - 10mM DTT - 1% Triton X-100 (Buffer A).
Then, a volume of 800µl of anti-proteases solution was added to this suspension (one tablet of a cocktail inhibitor marketed under the name Complete, Boehringer Mannheim, ref : 1836145) in 2ml of buffer A as well as a volume of 5ml of lysozyme solution (1mg in buffer A).

After a gentle shake, the mixture was incubated for 5 minutes on ice.

Lysis was completed by sonication on ice, three times 10 seconds with 10 seconds intervals with a microtip sonifier.

The lysate was clarified by centrifugation (5 min, +4°C, 20 000g). The surpernatant was withdrawn and can be stored at +4°C as such.

### 1.3.2 Purification

For 1L of bacterial culture, 2 ml of gel (Glutathione Sepharose 4B, Amersham Pharmacia Biotech, ref: 17-0756-01) was packed in a column of 1.6 cm internal diameter. This column was equilibrated with 5 column volume (cv) of buffer A, using a flow rate of 240cm/h. The column was loaded with the surpernatant at a flow rate of 42cm/h and then washed with 12cv of buffer A at a flow rate of 600cm/h. Finally, the GST-p47*phox*$SH3_{AB}$ was eluted by 3cv of buffer B at a flow rate of 240cm/h (buffer B containing 50mM Tris/HCl, pH 8, 5 mM glutathione. pH must be checked after the addition of glutathione).

### 1.3.3 Diafiltration and quantification

The elution that contained the purified p47*phox*$SH3_{AB}$, must be diafiltrated if protein quantification is necessary as free glutathione usually reacts with the protein assay. Diafiltration was performed on a membrane with a cut off of 10kDa (Centriprep, Amicon, ref : 4304). Successive dilutions with Hepes 0.1M, 0.4M KF allowed the decrease of the glutathione concentration below 8 µM. At this stage, proteins were quantified by the bicinchoninic acid micromethod (ref : P. K. Smith *et al*., *Anal. Biochem.* **150,** 76-85 (1985)). The purity of the preparation was checked by SDS-polyacrylamide gel electrophoresis using a silver nitrate staining method.

### Example 1 bis : Production of the proline-rich peptide p22*phox*.

The proline rich peptide was synthetised (SEQ ID N°3) and the purity was checked by sequence analysis and gel filtration (over 95%).

### Example 2 : The HTRF Assay

### 2.1 Materials and Methods

### 2.1.1. Preparation of the reagents

Assay mixtures were prepared in a final volume of 200 µl.

- *Buffer:* All the reagents are prepared in Hepes 100mM (Sigma H3375 PM 238.3), 50mM KF (Fluka 60238 PM 58.1), 0.1% BSA fraction V (Sigma A7906). The pH is adjusted to 8. Kept for 1 month at 4°C.
- *GST-p47phox*$SH3_{AB}$ (PM 40 000): Use of 50 µl of a 4X stock solution (200 nM). Final concentration is at 50 nM per well. Stable at 4°C up to 48h. Long term storage in the assay buffer at -20 or -80°C.
- *Peptide p22-(EuK)*$^{3+}$ (PM 3 200, [$Eu^{3+}$] = 0.0638 mg/ml) was synthesized by Neosystem, then labeled with Europium Cryptate and purified by Packard/CisBio international (ref C-28-E-K-004). The quantity of Europium cryptate used per well was 0.4 ng in order to obtain 40 000 counts (intensity of fluorescence) at 620 nm on the Discovery. Labeled peptide was stored at -20°C until needed.
  Use of 50 µl of a stock solution of 2.5 nM. Final concentration of 0.63 nM.
- Anti GST antibody was labeled with XL665 and purified by Packard/CisBio international (ref mAb GST-XI 009). The labeled antibody was stored at -80°C until needed. Stock solution was at 0.25 mg/ml and used at 400 ng/well (27.8 nM) in a volume of 96 µl. Final concentration of 13.3nM.

Table 1 :

| Compounds used respectively for positive and negative controls of the HTRF assay (The reagents concentrations indicated are the initial concentrations before adding to the assay). | | |
|---|---|---|
| | **Positive Control** | **Negative Control** |
| **Buffer** | 4 µl | 54 µl |
| **GST-p47*phox*$SH3_{AB}$ (200 nM)** | 50 µl | |
| **p22-EuK (2.5 nM)** | 50 µl | 50 µl |
| **Antibody-XL665 (27.8 nM)** | 96 µl | 96 µl |

### 2.1.2. HTRF reading

Assay mixtures were added to 96-well black plates (Packard Optiplate HTRF) (ref 6005207). No pre-incubation time was needed. HTRF data were collected using the Discovery® Microplate Analyzer specialized in HTRF (Packard's instruments). Excitation with a Nitrogen Laser is at 337 nm and fluorescence at both 620 et 665 nm is recorded simultaneously.
Curve fitting were accomplished using two Excel utility programs:

- Xlfit Wizard (model 200)
- Data analysis Wizard (model 68)

### 2.1.3 Calculation

For each well; of the microtiter plate, the following values are determined ::

- **Ratio**

Ratio (R) = signal 665nm / signal 620 nm X 10 000

- **Delta ratio**

Delta Ratio = Sample Ratio - Negative control ratio

This value represents the signal specific of the assay and is dependent of the apparatu

- **Delta F (expressed as %):**

Delta F = (Delta Ratio / Negative control ratio X 100

This value represents the specific signal minus the background signal and is independent of the apparatus.

### 2.2. Results

#### 2.2.1 Assay for binding of recombinant p47-*phox* SH3AB- to recombinant p22-*phox*-EuK.

A fixed final concentration of 80 nM of p47-*phox* SH3AB recombinant protein was added to the wells of a 96 well microtiter plate containing p22-*phox* EuK Then, a final concentration of anti-GST monoclonal antibody was added and the fluorescence signals et 620 nm and 665 nm were measured. The results are reported in Table 2 below;

Table 2 :

| Fluorescence measures of the binding of recombinant p47-*phox* SH3AB- to recombinant p22-*phox*-EuK. | | |
|---|---|---|
| | Positive Control | Negative Control |
| Ratio SD% | 2227 +/- 6.7% | 505 +/-2.7% |
| Signal noise Ratio | 4.4 | |

The fluorescence measures were repeated each hour, for a time period of 24 hours after the addition of the assay reagents.

Overall the signal was stable up to 4 h, with a slight decrease after 2 h. (Figure 3). Even though the signal was lower, readings were still possible after 24 hours.

#### 2.2.2. Titration of GST-p47-*phox*SH3$_{AB}$

- Final concentrations of GST-p47*phox*SH3$_{AB}$ were varied from 200 to 10 nM (from 4X stocks solutions) in a volume of 50 µl.
- p22-(EuK)$^{3+}$ peptide and anti GST antibody-XL665 were used at the final concentration of 0.63 nM and 13.3 nM respectively.

The results are shown in Figure 4.

The data showed that a maximum Delta F% ratio is reached for a final concentration of recombinant p47*phox*SH3$_{AB}$ of about 60 nM.

#### 2.2.3. Titration of the antibody antiGST-XL665

*Anti-GST-Xl665 antibody* concentrations were varied from 50 to 800 ng/well from a stock solution of 0.25 mg/ml in a volume of 96 µl. *GST-p47phoxSH3$_{AB}$ and p22-(EuK)$^{3+}$ peptide* were used at the final concentrations of 50 nM and 0.63 nM per well respectively.

The results are depicted in Figure 5.

The data presented in Figure 5 showed that the DeltaF% value increased with increasing amounts of the anti-GST recombinant antibody added to each well of the 96 well microtiter plate. Maximal Delta F% values were seen with 800 ng of anti-GST antibody per well.

### Example 3 : HTRF Assay - specificity of binding

#### 3.1. Mutation in GST-p47-*phox*SH3$_{AB}$

- *Mutated GST-p47-phox SH3$_{AB}$* : Final concentrations of GST-p47-*phox* SH3$_{AB}$ were varied from 200 to 10 nM (from a 4X stock solution) in a volume of 50 µl.

- *p22-(EuK)$^{3+}$ peptide* and *anti GST antibody-XL665* were used at identical final concentrations as previous experiment (0.63 nM and 13.3 nM respectively).

The results are depicted in Figure 6

The data showed that the recombinant mutated GST-p47*phox*$SH3_{AB}$ was totally incapable of binding to recombinant p22-$(EuK)^{3+}$ peptide, although this peptide bears a single amino acid substitution (156 P→ Q) as regards the wild type recombinant GST-p47*phox*$SH3_{AB}$.

**Example 4 : Assessing the screening assay with an inhibitor compound**

The screening assay was carried out with an inhibitor of binding consisting of the unlabeled recombinant p22-*phox* peptide.
The assay conditions were the following :

- *p22 peptide:* Final concentrations of p22 peptide were varied from 1 to 100 nM. Use of 4 µl of 50X stock solutions.
- *GST-p47-phoxSH3*$_{AB}$ and *p22-(EuK)*$^{3+}$ *peptide* and *anti GST antibody-XL665 XL665* were used at identical final concentrations as previous experiment (50 nM, 0.63 nM and 13.3 nM respectively).

Concentrations of the different reagents used in the assay are depicted in Table 3 hereunder.

Table 3 :

| Concentrations of the reagents used in the assay (The concentrations indicated are the initial concentrations of the reagents before addition to the assay) | | | | |
|---|---|---|---|---|
| | **Positive Control** | **Negative Control** | **Specificity Control** | **Competition with p22** |
| **Buffer** | 4 µl | 54 µl | 4 µl | |
| **GST-p47*phox*$SH3_{AB}$ (200 nM)** | 50 µl | | | 50 µl |
| **Mutated GST-p47*phox*$SH3_{AB}$ (200 nM)** | | | 50 µl | |
| **p22 (fr. 50 nM to 5µM)** | | | | 4 µl |
| **p22-EuK (2.5 nM)** | 50 µl | 50 µl | 50 µl | 50 µl |
| **Antibody-XL665 (27.8 nM)** | 96 µl | 96 µl | 96 µl | 96 µl |

The results are depicted in Figure 7A and 7B.

The data presented in Figure 7A (left side of Figure 7) showed that the competitive inhibitory effect of the unlabeled p22 peptide on the binding of p22 EuK on p47*phox*-GST is concentration dependant.

The plotting of the inhibition percentage values for each unlabeled p22 final concentration in the assay allow the calculation of the IC50 value, which was 5.8 nM/ (Figure 7B).

**Example 5 : Large scale screening of candidate inhibitor compounds with the screening method of the invention.**

**A. Materials and Methods**

**A.1 Screening assay**

- *Candidate compounds tested:* Tested at 20µM in 0.2% DMSO. Used of 4 µl of the products at 1 mM stock concentration.

- *GST-p47phoxSH3$_{AB}$*, *p22-(EuK)$^{3+}$ peptide* and *anti GST antibody-XL665* were used at identical final concentrations as previous experiment (50 nM, 0.63 nM and 13.3 nM respectively).
- *p22 peptide:* Preparation of a stock solution at 500 nM. Used at a concentration just above its IC50 (10 nM in 4µl) as an internal control of inhibition to validate each plate.

The order of addition of the reagents in the plate were:

1. Buffer/p22 peptide/candidate compounds
2. GST-p47phoxSH3$_{AB}$
3. p22-(EuK)$^{3+}$ peptide
4. anti GST antibody-XL665

Positive and negative controls were tested in 7 wells each. The candidate compounds and the p22 peptide internal control were tested in duplicate. For each, the mean value ± Cv was calculated.

**A.2 Determination of the IC50**

*Candidate compounds:* Final concentration varied from 30 à 0.04 µM. Use of 4 µl.of 50X stock solutions ranging from 1.5 mM à 2 µM. *GST-p47-phoxSH3$_{AB}$* , *p22-(EuK)$^{3+}$ peptide* and *anti GST antibody-XL665* were used at identical final concentrations as previous experiment (50 nM, 0.63 nM and 13.3 nM respectively).

Table 4 :

| Concentrations of the different reagents in the assay (Indicated concentrations are the initial concentration of the reagents which are used in definite amounts in the assay) | | | | | |
|---|---|---|---|---|---|
| | **Positive Control** | **Negative Control** | **Inhibition Control (Ic50 of p22)** | **Test Screening** | **IC50 Determi nation** |
| **Buffer** | 4 µl | 54 µl | | | |
| **Candidate compounds (1 mM)** | | | | 4 µl | |
| **Candidate compounds (2 µM à 1.5 mM )** | | | | | 4 µl |
| **GST-p47*phox*SH3$_{AB}$ (200 nM)** | 50 µl | | 50 µl | 50 µl | 50 µl |
| **p22 (500 nM)** | | | 4 µl | | |
| **p22-EuK (2.5 nM)** | 50 µl | 50 µl | 50 µl | 50 µl | 50 µl |
| **Antibody-XL665 (27.8 nM)** | 96 µl | 96 µl | 96 µl | 96 µl | 96 µl |

### A.3 Cell-free assay for NADPH oxidase :

The assay used is derived from that disclosed by ABO et al. (1995).

Superoxide generation assays were performed in 96-well plates in a final volume of 100µL at pH 8 comprising: 2µL of compound dissolved in DMSO at the desired concentration, 0.4µg PLB985 membranes, 0.2µg recombinant p47$^{phox}$, 0.1 µg recombinant p67$^{phox}$, 0.15µg Rac1-GTPγS loaded, 10µM FAD, 100µM ferricytochrome c, 65mM NaP04, 1mM MgCl2, 1mM EGTA. Assembly process was triggered by addition of 25µL LiDS (80µM final concentration) at room temperature. Reaction was started 5 min later by addition of 25µL NADPH (200µM final concentration). Superoxide generation was measured by following the rate of superoxide dismutase inhibitable reduction of ferricytochrome c at 550 nM in a Labsystem iEMS Reader MF. The IC50 measured is defined as the concentration of inhibitor that divides the enzyme initial velocity by 2.

| | |
|---|---|
| Neutrophil membranes: | prepared from PLB 985 cells by Onyx. Stored in 120mM $NaP0_4$ pH 7.4, 1mM EGTA, 1mM $MgCl_2$, 1mM DTT, 1mM leupeptin, 1mM PMSF, 20% glycerol, 8mM γ-D octyglucoside. Stable for 1 thaw and refreeze cycle with storage at -80°C |
| Rac1-GTPγ S (EE tag) : | prepared and purified by Onyx. Stored in 25mM Tris, pH7.5, 25mM NaCl, 7.5mM $MgCl_2$, 5mM EDTA, 50% glycerol. Stable, store at -80°C in small aliquots to avoid freeze /thaw cycles. |
| p47-*phox* (EE tag) | prepared and purified by Onyx. Stored in 25mM Tris, pH7.5, 1mM EDTA, 50% glycerol. Stable, store at -80°C. |
| p67-*phox* (EE tag) | prepared and purified by Onyx. Stored in 25mM Tris, pH7.5, 1mM EDTA, 50% glycerol. Stable, store at - 80°C. |

### A.4 Secondary HL60 cell based assay for NADPH oxidase

**Protocol: NADPH oxidase measurement in HL60 cells differentiated in neutrophils**

#### 1. Materials

RPMI medium, Hank's Balanced Salt solution (HBSS), fetal calf serum, L-glutamine, gentamycin, PBS and Hepes were from Gibco/BRL. DMSO was from Merck. Cytochalasin, TPA and cytochrome C were from Sigma.

#### 2. Methods

#### 2.1 Cell Culture and differentiation

HL-60 cells were obtained from the European Collection of cell Culture (ECACC). They were grown in RPMI medium supplemented with 10% (v/v) heat-inactivated calf serum at 37°C in an humidified atmosphere of 95% air- 5% $CO_2$. The culture medium was changed every 2 to 3 days. For differentiation, HL-60 cells were seeded at 250,000-400,000 cells/ml and 1.3% DMSO was added to the medium for 7 to 10 days. During the differentiation process the culture medium was not changed and the cells were ready in 7-10 days and were approximately at 1,000,000 cells/ml.

#### 2.2 Assay

HL-60 cells differentiated in neutrophils were used at a concentration of $1.2x10^6$ cells/ml in Hank's + 120µM cytochrome C and 240µl was distributed in each well of a 96 wells plate and incubated for 5 min at room temperature. 240 µL of the cellular suspension was then distributed in a 96-wells plate already containing the compounds to test diluted in 30 µl of DMSO at the chosen concentration. Cells were incubated in the presence of the compounds for 30 min at 37°C and the optical density of the suspension was continuously monitored at 550 nm. 30 µL of 1 µM TPA Each compound to test at different concentration (10µl max/well in DMSO 1/10) was incubated 30min with the cells at 37°C under shaking before addition of 100nM PMA for 30-40min. In the negative control no compound and no PMA was added, in the positive control no compound was added but the cells were stimulated with PMA (0% inhibition). The absorbance in each well was monitored at 550nm.

The activity of the compounds was expressed as a percentage of inhibition of NADPH oxidase activity compared to the vehicle treated cells.

The percentage of inhibition was calculated as :

$$\% \text{ inhibition} = 100 - \left[ \frac{X - T^-}{T^+ - T^-} \ x\ 100 \right]$$

With $X$: OD in well for each concentration of compound tested
With $T^-$ : OD in negative control well (no stimulation)
With $T^+$: OD in positive control well (0% inhibition)

**B. Results**

Various compounds belonging to the coumarine or the quinazoline families were tested using the screening method of the invention. A comparison of the IC50 values obtained according to the screening method of the invention and the IC50 values obtained with assays (cell free and cell-based assays) revealing the inhibition of the NADPH oxidase activity was performed, in order to assess the biological relevance of the HTRF assay of the invention. The results are depicted in Table 6 below.

Table 6 :

Comparison between the IC50 values obtained with the screening method of the invention and the IC50 values obtained with conventional assays.

| | | **IC50 values (µM)** | |
|---|---|---|---|
| **Compounds** | **HTRF Assay** | **Cell-free assay** | **HL60 assay** |
| Coumarine A | 10 | 3.74 | >30 |
| Coumarine B | 15 | 10 | 2.06 |
| Coumarine C | 16 | 61 | >30 |
| Quinazoline A | 6 | 53.4 | NT* |
| Quinazoline B | 12 | 5.1 | 1.39 |

The results shown in Table 6 above demonstrate that the inhibition of binding between GST-p47*phox*$SH3_{AB}$ and p22-$(EuK)^{3+}$ peptide detected according to the screening method of the invention always correspond to compounds that effectively inhibit the NADPH oxidase enzyme activity, such as illustrated by the inhibitory activity of these compounds detected according to the cell-free and cell-based NADPH oxidase assay.

**Example 6 : High Throughput screening assay**

**A. Materials and Methods**

**A.1 Screening assay**

Assay mixtures were prepared in a final volume of 50 µl.

- *Buffer:* identical to 96 well format.
- *GST-p47phoxSH3$_{AB}$* : Use of 12 µl of a 4.167X stock solution (208 nM). Final concentration is at 50 nM per well.
- *p22-(EuK)$^{3+}$ peptide:* The quantity of Europium cryptate used per well was 1.6 ng in order to obtain 40 000 counts (intensity of fluorescence) at 620 nm on the Discovery. Use of 12 µl of a stock solution at 41.67 nM. Final concentration of 10 nM.
- Anti GST antibody: Stock solution was at 0.25 mg/ml and used at 400 ng/well in a volume of 24 µl. Final concentration of 53.3 nM.

- *Candidate compounds:* Tested at 20µM in 0.2% DMSO. Used of 1 µl of the products at 1 mM stock concentration.

**A.2 Determination of the IC50**

- *Candidate Compounds:* Use of 1 µl of 50X stock solutions ranging from 1.5 mM à 2 µM.
- *GST-p47-phoxSH3$_{AB}$ , p22-(EuK)$^{3+}$ peptide* and *anti GST antibody-XL665* were used at identical final concentrations as previous experiment (50 nM, 10 nM and 53.3 nM respectively).

**B. Results : Validation of the 384-well HTRF assay format**

**B.1 Comparartive analysis between 96 well and 384 well assays**

A comparison between the Signal/Noise ratios obtained with the 96 well assay described in the examples above and the high throughput 384 well assay was performed. The results are depicted in Table 5 below.

**Table 5 :** Comparison of the Signal/Noise ratios in 96 well and in high throughput 384 well inhibitor screening method of the invention.

**B.2 Comparison of inhibiting properties of different compounds between 96 well and 384 well assays at different peptide-EuK concentrations**

The percentage of inhibition of binding between GST-p47-*phox*SH3$_{AB}$

| 40 nM GST-p47phoxSH3$_{AB}$ | | 96 wells | 384 wells EuK 0.4ng/well | 384 wells EuK 0.8ng/well | 384 well s EuK 1.6ng/well |
|---|---|---|---|---|---|
| Négative Control | Ratio | 533 | 667 | 513 | 440 |
| | SD% | +/-6 | +/-7 | +/-6 | +/-3 |
| Positive Control | Ratio | 1859 | 1446 | 1304 | 1208 |
| | SD% | +/-1 | +/-2 | +/-2 | +/-1 |
| Signal/noise Ratio | | 3.5 | 2.2 | 2.6 | 2.8 |

and p22-(EuK)$^{3+}$ for three different candidate inhibitor compounds was carried out. The results are depicted in Figure 8. The data depicted in Figure 8 showed that practically identical percentage of inhibition values are obtained using the 96 well assay and the 384 well assay. Additionally, approximately same values are obtained for amounts of p22-(EuK)$^{3+}$peptide increasing from 0.4 to 1.6 ng/well.

**REFERENCES:**

- **ABO A. et al**., 1995, Methods in Enzymology, vol.256: 268-278
- **AGHAZADEAH et al**., 1999, Chem. Biol. vol.6: R241
- **BOOKER GW et al**., 1993, Cell., vol.73:813-822.
- **BORCHERT, 1994**, FEBS. Letters, vol.341:79-85.
- **BLANCO PJ et al**., 1997, J.Biomol. NMR, vol.9:347-357.
- **CHARDIN P et al.,** 1993, Science, vol.260:1338-1343
- **CHEN JK et al. 1993**, J. Am. Chem. Soc., vol.115:12.591-12.592,
- **CHERNIACK AD et al**; 1994, J. Biol. Chem. Vol.269: 4717-4720.
- **DALY RJ et al;** 1994, Oncogene, vol.9:2723-2727.
- **DARNELL JE et al.** 1994, Science, vol. 264:1415-1421.

- **DE MENDEZ I. et al**., 1994, J. Biol. Chem, 269:16326.
- **(DE MENDEZ I. et al**., 1997, Mol. and cell.Biol. , vol.17 (4): 2177-2185.
- **DE WEERSE M. et al.,** 1994, J. Biol. Chem. , vol.269:23857-23860.
- **DALGARNO DC et al.** 1998, Biopolymers, vol.43(5): 383-400,
- **FORSTER T,** 1948, Ann. Physik. (Leipzig) vol.2: 55-751949).
- **FORSTER T.,** 1959, Faraday Soc. dis., vol.27: 1-17.
- **FENG S. et al.** 1994, Sci., vol.266: 1241-1246.
- **GORINA S. et al.** 1996, Science, vol.274:1001-1005.
- **GOSSER YQ et al**., 1995, Structure, vol.3:1075-1086.
- **GOTTESMAN MM** J. Natl. Cancer Inst., vol.86:1277-1285
- **GURUPRASAD L.** et al., J. Mol. Biol. vol. 248:856-866.
- **HIROAKI H. et al.,** 1996, J.Biomol. NMR, vol.8:105-122.
- **HU Q,** 1995, Mol. Cell. Biol., vol.15:1169-1174.
- **IHLE IN ,** 1994, Proc. Soc. Exp. Biol. Med., vol.206:268-272.
- **JAMES PW et al.** 1994, Oncogene, vol.9:3601-3608.
- **JHUN BH et al.,** 1994, Mol.Cell. Biol., vol.14: 7466-7475.
- **KISHAN KVR et al.,** 1997, Nat. Struct. Biol. , vol.4:739-743.
- **KOYAMA S et al.** 1993, Cell, vol.72: 949-952.
- **KOHDA D. et al.** 1993, Cell., vol.72: 953-960.
- **KOHDA D. et al. ,** 1994, Structure, vol.2:1029-1040.
- **(LETO TL et al,** 1994, Proc. Natl. Acad. Sci. USA, vol.91:10.650-10.654).
- **LIANG J et al.,** 1996, J.Mol. Biol., vol.257:632-643.
- **LI N et al. ,** 1993, Nature (London), vol.363:85-88.
- **LOWE C et al.,** 1993, Proc. Natl. Acad. Sci. USA, vol.90:4485-4489.
- **LUTTRELL DK et al.,** 1994, Proc. Natl. Acad. Sci. USA, vol.91: 83-87.
- **LIM WA. et al. ,** 1994, Nature, (London), vol.372: 375-379.
- **MAIGNAN S et al.,** 1995, Science, Vol.268:291-293.
- **MAYER BJ and ECK MJ ,** 1995 , Current. Biol., vol.5 : 364-367.
- **MERRIFIELD RB,** 1965a, Nature, 207 (1996): 522-523.
- **MERRIFIELD RB,** 1965b, Science, 150 (693): 178-185.
- **MORTON CJ et al.,** 1996, Structure, vol.4:705-714.
- **MOODIE SA et al.,** 1994, Mol. Cell Biol., vol.14:7153-7162
- **MUSACCHIO A. et al.** 1994, Nature Struct. Biol. , vol.1:546-551.
- **MUSACCHIO A et al. ,** 1992, Nature (London), Vol.359: 851-855
- **NAM HJ et al.,** 1996, Structure, vol.4: 1105-1114.
- **NOBLE et al.,** 1993, EMBO J., vol.12: 2617-2624.
- **ODA T. et al.,** 1994, J. Biokl. Chem., vol.269:22.925-22.958.
- **PAWSON T,** 1995, Nature, Vol.373: 573-580.
- **PENDERGAST AM et al. ,** 1993, Cell, vol.75:175-185.
- **REN R. et al.** 1993, Sci. vol.259: 1157-1161.
- **RAVICHANDRAN KS et al.,** 1995, Mol. Cell Biol. , vol.15: 593-600.
- **RICKLES R. et al.** 1994, EMBO J. , vol.13:5.598-5604,
- **SAMBROOK J. FRITSCH E.F. and T. MANIATIS,** 1989. Molecular cloning: A laboratory manual, 2 ed. Cold Spring Harbor Laboratory, Cold Spring Harbor, New York.
- **SATO K et al. ,** 1994, Leukemia, vol.8: 1663-1672
- **SAKSELA K et al.,** 1995, EMBO J. vol.14:484-491.
- **SUMIMOTO H. et al.** 1996, J. Biol. Chem. , vol.271(36):22152-22158.
- **WITTKIND M. et al.,** 1994, Biochemistry, vol.33: 13531-13539
- **YANG YS. et al.,** 1994, EMBO J. , vol.13:1270-1279.
- **YAMACHITA T. et al.,** 1994, Proc. Natl. Acad. Sci. USA, vol.91:11.251-11.255.
- **YU H. et al.,** 1994, Cell, vol.76: 933-945.
- **YU H. et al.** 1992, Sci. vol.258:1665-1668.
- **ZHANG P. et al.,** 1995, Structure, vol.3:1185-1195.
- **CESARENI et al.,** 2002, FEBS Letters, vol.513:38-44
- **KEFALAS et al.,** 1995, Int. J. Biochem. Cell. Biol., vol. 27(6):551-63
- **LIM et al.,** 1996, Structure, vol. 4(6):657-9

**Claims**

**1.** A method for the screening of a candidate compound for inhibiting the interaction between a proline-rich peptide and a SH3 domain-comprising peptide by Homogeneous Time-Resolved Fluorescence technique, said method comprising the steps of:

a) providing a buffer solution containing a proline-rich peptide which is labelled with a first fluorescent marker, wherein said first fluorescent marker is the first partner or the second partner of paired FRET fluorescence markers;
b) adding the candidate compound to the solution obtained at step a);
c) adding the SH3 domain-comprising peptide which is directly or indirectly labelled with a second fluorescent marker and wherein said second fluorescent marker is the second partner or the first partner of paired FRET fluorescence markers;
d) submitting the mixture obtained at step d) to a source of energy at a wavelength corresponding to the excitation wavelength of the first partner of paired FRET fluorescence markers and measuring the fluorescence signal at the emission wavelength of the second partner of paired FRET fluorescence markers;
e) comparing the fluorescence signal value obtained at step e) with the fluorescence signal value obtained when step b) is omitted to determine if the candidate compound inhibits the interaction between the proline-rich peptide and the SH3 domain-comprising peptide.

**2.** The method of claim 1, wherein the first fluorescent marker is the first partner and the second fluorescent marker is the second partner of paired FRET fluorescent markers.

**3.** The method of claim 1, wherein the first fluorescent marker is the second partner and the second fluorescent marker is the first partner of paired FRET fluorescent markers.

**4.** A method according to anyone of claims 1 to 3, wherein the first partner of paired FRET fluorescent markers is Europium cryptate.

**5.** A method according to anyone of claims 1 to 4, wherein the second partner of paired FRET fluorescent markers is XL665.

**6.** A method according to anyone of claims 1 to 5, wherein the SH3 domain-comprising peptide is indirectly labelled with a second fluorescent marker, in which case said SH3 domain-comprising peptide is covalently bound to a detectable molecule having specific affinity for a labelling reagent comprising the second fluorescent marker, said labelling reagent being added during step c), subsequently to the addition of the SH3 domain-comprising peptide.

**7.** The method of claim 6, wherein the detectable molecule is a Glutathion S Tranferase (GST) protein.

**8.** The method of claim 6, wherein the labelling reagent is an antibody directed to the GST protein which is labelled with the second fluorescent marker.

**9.** A method according to anyone of claims 1 to 8, wherein the proline-rich peptide is selected from the group consisting of the proline-rich peptides contained in the Fyn, Abl, CrkN, Src, Nsrc, PIK and Nef proteins.

**10.** A method according to anyone of claims 1 to 8, wherein the proline-rich peptide is selected from the group of proline-rich peptides contained in the p22phox, p47phox and p67phox sub-unit proteins of the human NADPH oxidase.

**11.** A method according to anyone of claims 1 to 8, wherein the proline-rich peptide is a proline-rich peptide contained in the p22phox, sub-unit protein of the human NADPH oxidase.

**12.** A Method according to anyone of claims 1 to 11, wherein the proline rich peptide comprises a proline rich amino acid sequence of approximately 10 amino acids in length which binds to an SH3 domain with a dissociation constant comprised between 5 and 100 µM.

**13.** A Method according to anyone of claims 1 to 12, wherein the proline rich peptide comprises a proline rich amino acid sequence selected from « Pro-X-X-Pro », « +-X-X-Pro-X-X-X-Pro », « Pro-X-X-Pro-X-+ », « +-X-q-Pro-X-q-Pro » and « q-Pro-X-q-Pro-X-+ », wherein X denotes any aminoacid residue, + denotes Arg or Lys and q denotes a hydrophobic aminoacid.

**14.** A Method according to anyone of claims 1 to 12, wherein the proline rich peptide comprises a proline rich amino acid sequence selected from « R-X-#-Pro-X-X-Pro », « -Pro-X-X-Pro-X-R », « +-X-X-Pro-X-X-Pro», «Pro-X-X-Pro-X-+» , « Pro-X-@-X-X-Pro-X-X-Pro» and « Pro-X-X-D-Y», wherein X denotes any aminoacid residue, + denotes Arg or Lys, @ denotes an aromatic or aliphatic aminoacid residue and # denotes an aromatic aminoacid residue.

**15.** A method according to anyone of claims 11 to 14, wherein the proline-rich peptide comprises at least 10 consecutive proline rich amino acids from the amino acid sequence of SEQ ID N°3.

**16.** A method according to anyone of claims 11 to 15, wherein the proline-rich peptide consists of the amino acid sequence of SEQ ID N°3.

**17.** A method according to anyone of claims 1 to 16 , wherein the proline-rich peptide has from 10 to 100 amino acids in length.

**18.** A method according to anyone of claims 1 to 17, wherein the SH3 domain comprising peptide has from 55 to 100 amino acids in length.

**19.** A method according to anyone of claims 1 to 18, wherein the SH3 domain of the SH3 domain-comprising peptide is selected from the group of SH3 domains contained in human PI3K p85, mouse Abl, human Fyn, human c-Src, human Lck, C elegans SEM-5, mouse Grb2, human Grb2, mouse c-Crk, human 53BP2 and human Hck kinase proteins.

**20.** A method according to anyone of claims 1 to 18, wherein the SH3 domain of the SH3 domain-comprising peptide is selected from the group of SH3 domains contained in human PI3K p85, human Abl, human Fyn, human c-Src, human Lck, human Lyn, human Grb2, human PLC-γ, human 53BP2 human GAP, human 53BP2, human Csk and human Hck kinase proteins.

**21.** A method according to anyone of claims 1 to 18, wherein the SH3 domain of the SH3 domain-comprising peptide is selected from the group of SH3 domains contained in the p47phox and p67phox sub-unit proteins of the human NADPH oxidase.

**22.** A method according to anyone of claims 1 to 18, wherein the SH3 domain of the SH3 domain-comprising peptide is an SH3 domain contained in the p47phox sub-unit protein of the human NADPH oxidase.

**23.** The method of claim 22, wherein the SH3 domain-comprising peptide comprises the amino acid sequence of SEQ ID N°4.

**24.** A screening reagent consisting of a proline-rich peptide which is labelled with a first fluorescent marker, wherein said first fluorescent marker is the first partner or the second partner of paired FRET fluorescence markers.

**25.** The screening reagent of claim 24, wherein the first fluorescent marker is a Europium cryptate molecule.

**26.** A screening reagent according to anyone of claims 24 to 25 consisting of the proline-rich peptide having the amino acid sequence of SEQ ID N°3 which is covalently bound to a Europium cryptate molecule.

**27.** A screening reagent consisting of a SH3 domain-comprising peptide which is directly or indirectly labelled with a second fluorescent marker and wherein said second fluorescent marker is the second partner or the first partner of paired FRET fluorescence markers.

**28.** The screening reagent of claim 27, wherein the second fluorescent marker is XL665.

**29.** A kit for the screening of a candidate compound for inhibiting the interaction between a proline-rich peptide and a SH3 domain-comprising peptide comprising:

a) a first screening reagent consisting of a proline-rich peptide which is labelled with a first fluorescence marker, wherein said first fluorescent marker is the first partner or the second partner of paired FRET fluorescence markers;

b) a second screening reagent consisting of a SH3 domain-comprising peptide which is directly or indirectly labelled with a second fluorescent marker and wherein said fluorescent marker is the second partner or the first partner of paired FRET fluorescence markers.

**30.** A screening reagent according to anyone of claims 27 to 28, consisting of the SH3 domain-comprising peptide having the amino acid sequence SEQ ID N°4 which is fused to a GST protein.

**31.** A complex formed between :

a) a proline-rich peptide which is labelled with a first fluorescent marker, wherein said first fluorescent marker is the first partner or the second partner of paired FRET fluorescence markers; and

b) a SH3 domain-comprising peptide which is directly or indirectly labelled with a second fluorescent marker and wherein said second fluorescent marker is the second partner or the first partner of paired FRET fluorescence markers.

**32.** A complex formed between :

a) a proline-rich peptide which is labelled with a first fluorescent marker, wherein said first fluorescent marker is the first partner or the second partner of paired FRET fluorescence markers; and

b) a candidate compound which inhibits the interaction between the proline-rich peptide defined in a) and a SH3 domain-comprising peptide.

**33.** A complex formed between :

a) a SH3 domain-comprising peptide which is directly or indirectly labelled with a second fluorescent marker and wherein said second fluorescent marker is the second partner or the first partner of paired FRET fluorescence markers; and

b) a candidate compound which inhibits the interaction between a proline-rich peptide and the SH3 domain-comprising peptide defined in a).

**34.** The use of a candidate compound selected according to a method according to anyone of claims 1 to 23 for manufacturing a pharmaceutical composition.

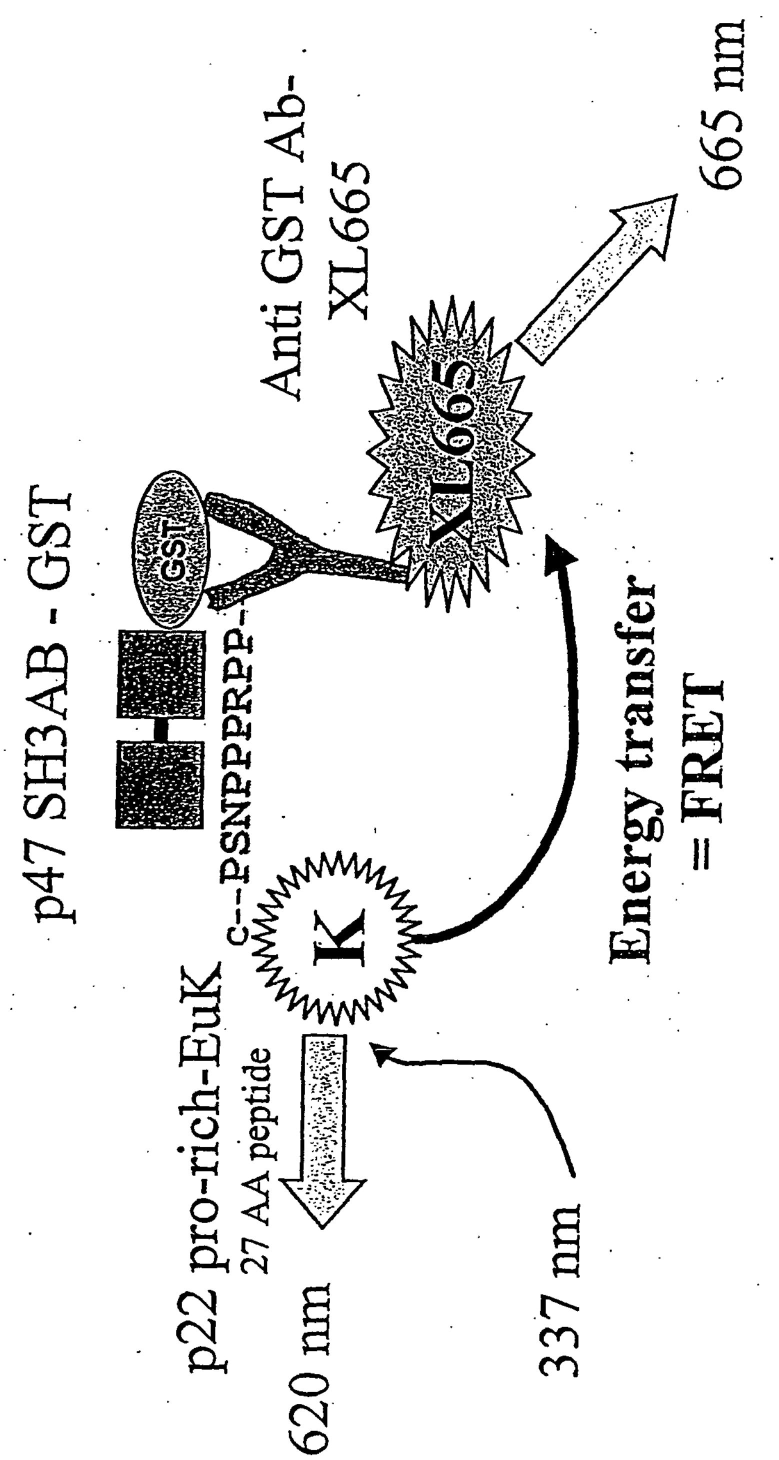
p47 SH3AB - GST
GST
Anti GST Ab-
XL665
XL665
665 nm
p22 pro-rich-EuK
27 AA peptide
C--PSNPPPRPP-
K
620 nm
337 nm
Energy transfer
= FRET

FIGURE 1

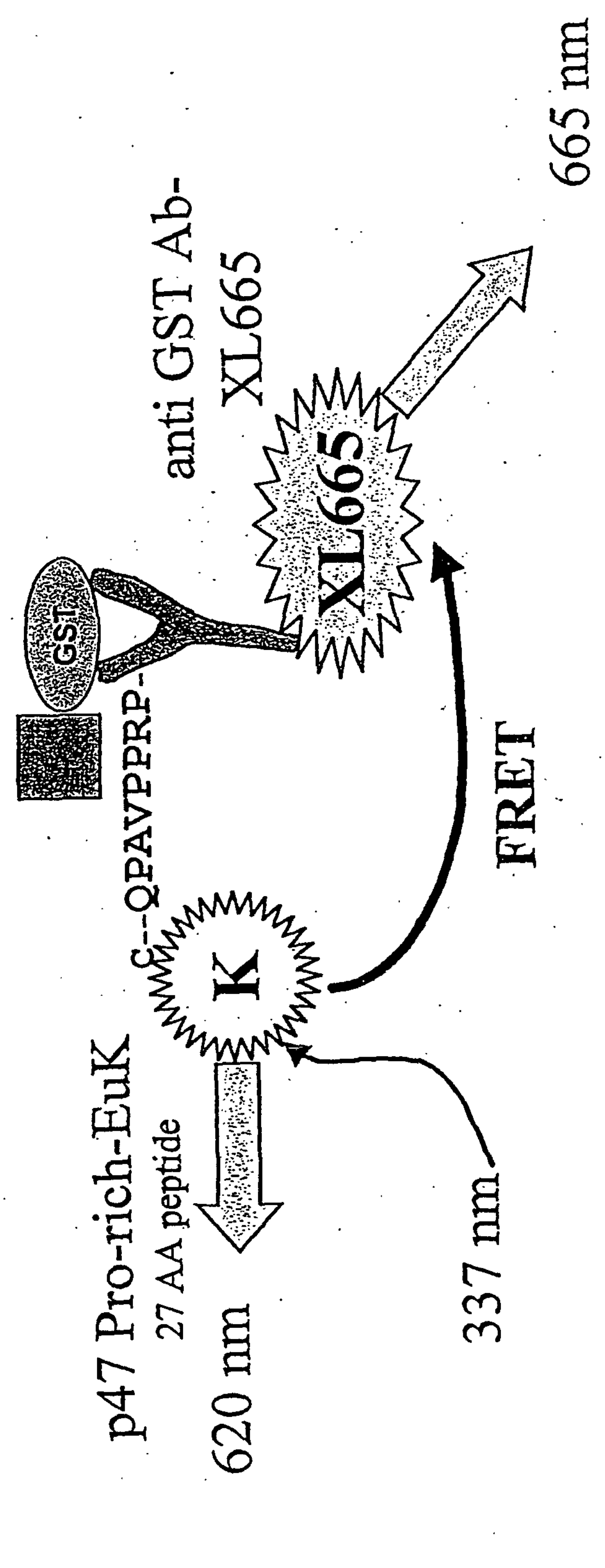
p67 SH3B - GST
GST
anti GST Ab-
XL665
XL665
665 nm
ε--QPAVPPRP-
K
FRET
p47 Pro-rich-EuK
27 AA peptide
620 nm
337 nm

FIGURE 2

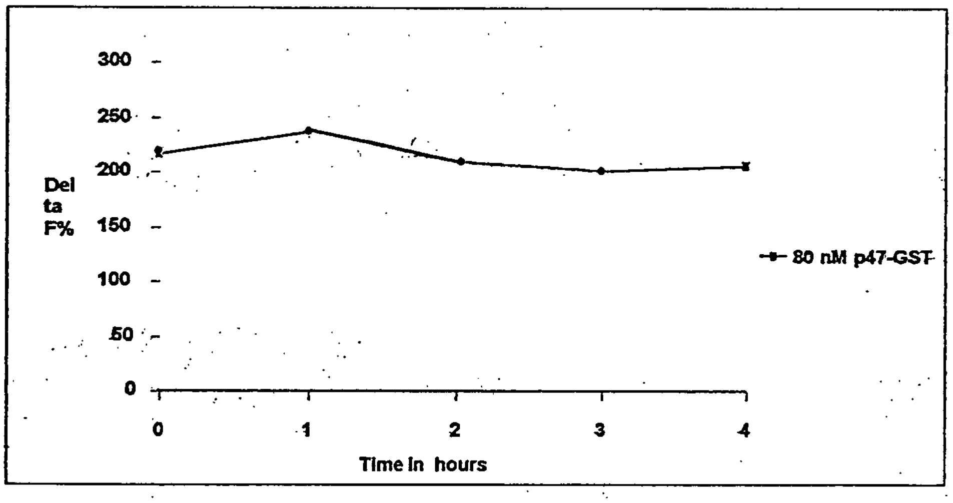
300
250
200
150
100
50
0
Del
ta
F%
0
1
2
3
4
Time in hours
80 nM p47-GST

Figure 3

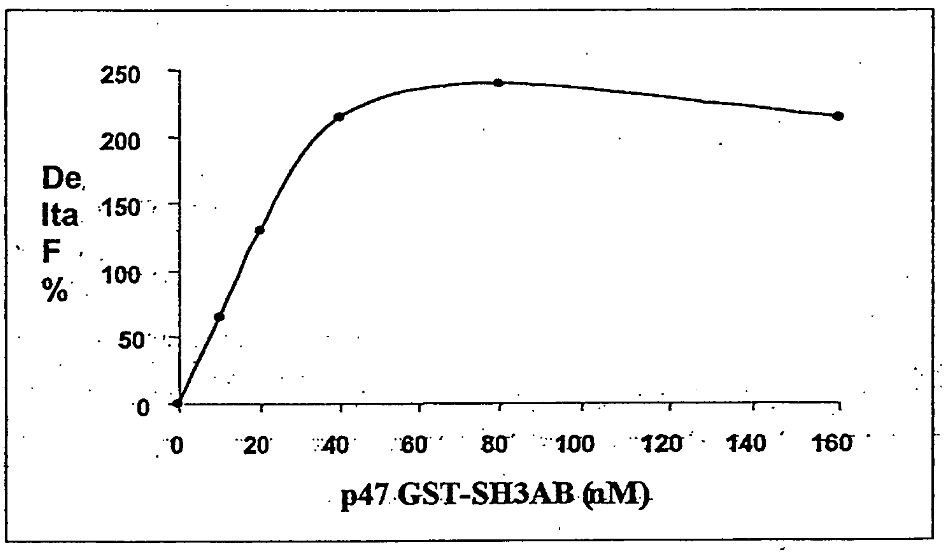

250
200
150
100
50
0
Delta F %
0
20
40
60
80
100
120
140
160
p47 GST-SH3AB (nM)


Figure 4

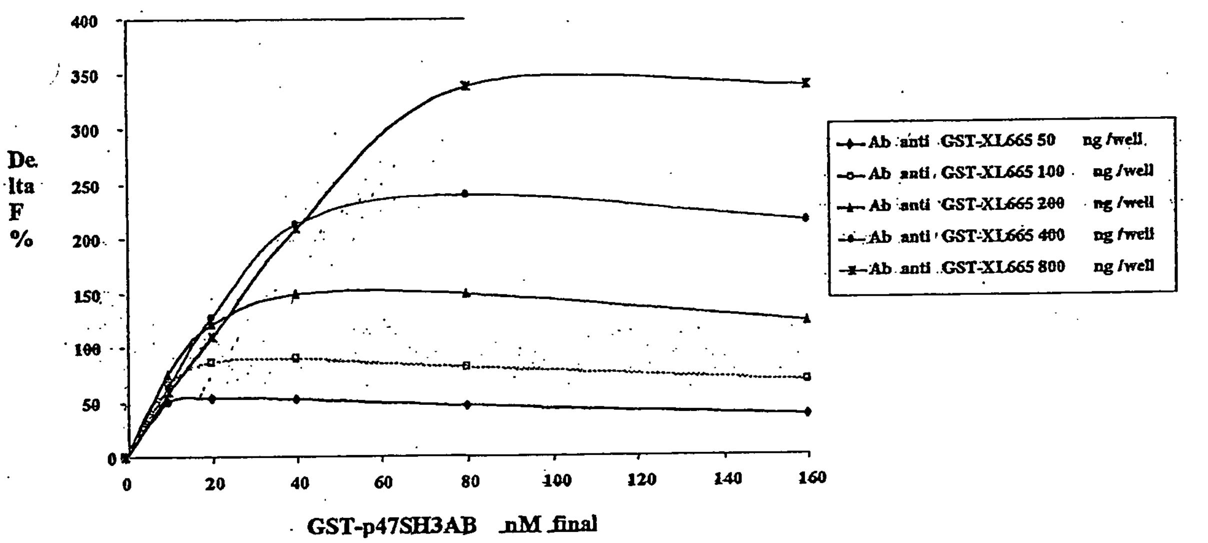
400
350
300
250
200
150
100
50
0
Delta F %
0
20
40
60
80
100
120
140
160
GST-p47SH3AB nM final
Ab anti GST-XL665 50 ng /well
Ab anti GST-XL665 100 ng /well
Ab anti GST-XL665 200 ng /well
Ab anti GST-XL665 400 ng /well
Ab anti GST-XL665 800 ng /well

**Figure 5**

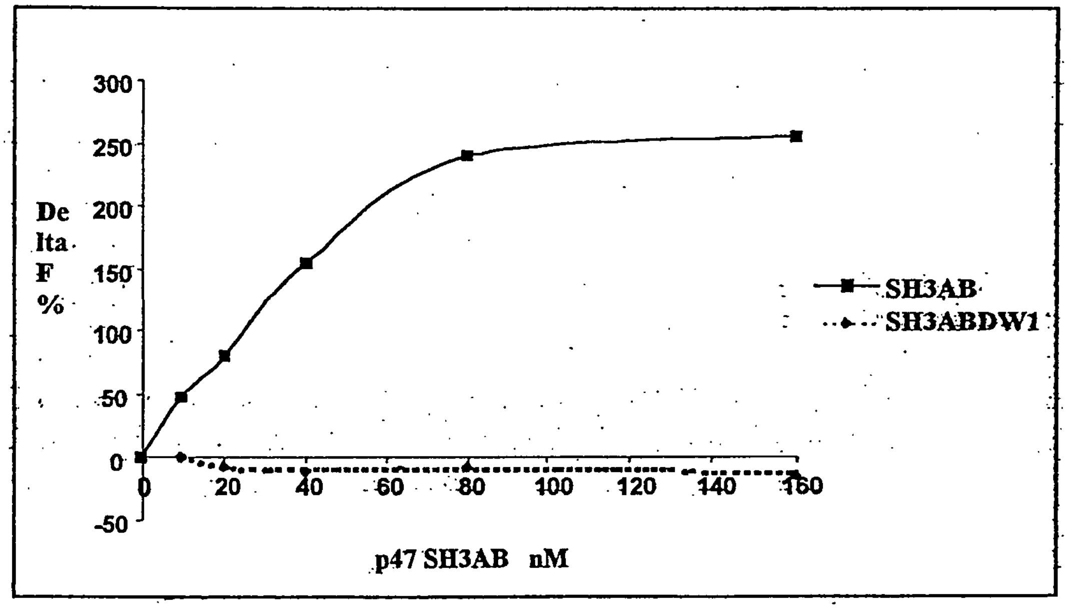
300
250
200
150
100
50
0
-50
De
lta
F
%
0
20
40
60
80
100
120
140
160
p47 SH3AB nM
SH3AB
SH3ABDW1

**Figure 6**

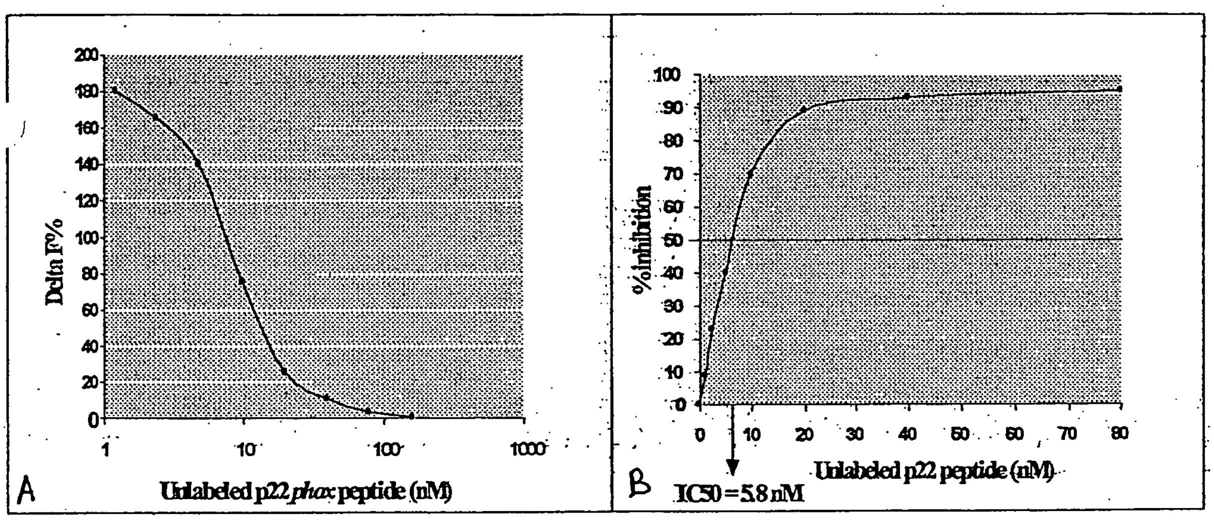
200
180
160
140
120
100
80
60
40
20
0
Delta P%
1
10
100
1000
A
Unlabeled p22*phox* peptide (nM)
100
90
80
70
60
50
40
30
20
10
0
% inhibition
0
10
20
30
40
50
60
70
80
Unlabeled p22 peptide (nM)
B
IC50 = 5.8 nM

**Figure 7**

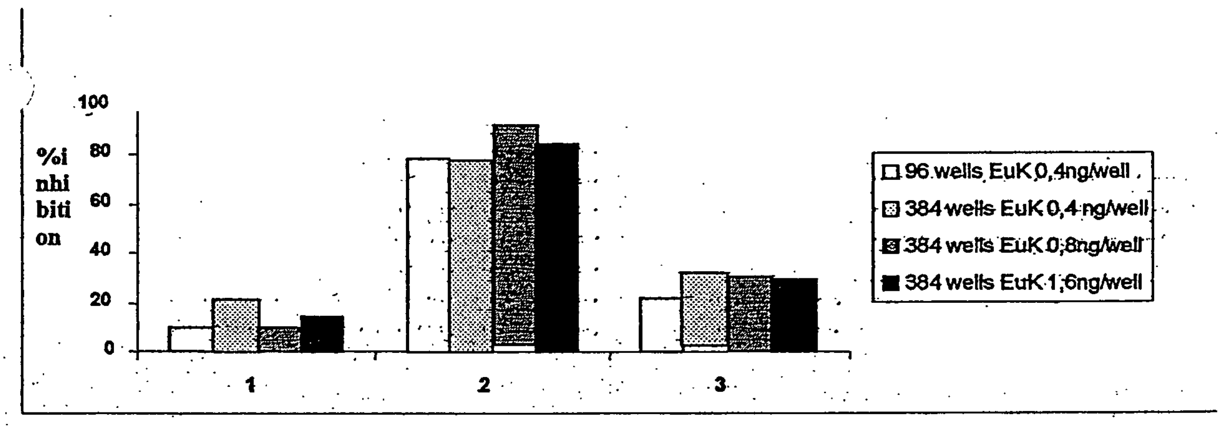
%inhibition
100
80
60
40
20
0
1
2
3
96 wells EuK 0,4ng/well
384 wells EuK 0,4 ng/well
384 wells EuK 0,8ng/well
384 wells EuK 1,6ng/well

Figure 8